(19) Europäisches Patentamt
European Patent Office
Office européen des brevets

(11) Publication number: **0 685 467 A1**

(12) # EUROPEAN PATENT APPLICATION published in accordance with Art. 158(3) EPC

(21) Application number: **95902967.9**

(22) Date of filing: **16.12.94**

(86) International application number: **PCT/JP94/02114**

(87) International publication number: **WO 95/16677 (22.06.95 95/26)**

(51) Int. Cl.[6]: **C07D 239/34**, C07D 239/36, C07D 239/38, C07D 239/40, C07D 239/42, C07D 403/12, A61K 31/505

(30) Priority: **16.12.93 JP 343612/93**

(43) Date of publication of application: **06.12.95 Bulletin 95/49**

(84) Designated Contracting States: **DE GB**

(71) Applicant: **NKK CORPORATION**
**1-2-1 Chome, Marunouchi**
**Chiyoda-ku**
**Tokyo 100 (JP)**

(72) Inventor: **OHKUBO, Akihiro**
**NKK Corporation**
**1-2, Marunouchi 1-chome**
**Chiyoda-ku**
**Tokyo 100 (JP)**
Inventor: **MINEGISHI, Torataro**
**NKK Corporation**
**1-2, Marunouchi 1-chome**
**Chiyoda-ku**
**Tokyo 100 (JP)**
Inventor: **MIZUTA, Tadashi**
**NKK Corporation**
**1-2, Marunouchi 1-chome**
**Chiyoda-ku**
**Tokyo 100 (JP)**
Inventor: **SATO, Hiroaki**
**NKK Corporation**
**1-2, Marunouchi 1-chome**
**Chiyoda-ku**
**Tokyo 100 (JP)**
Inventor: **YAMADA, Masashi**
**8-2-3, Mizukino**
**Moriya-machi**
**Kitasoma-gun**
**Ibaraki 302-01 (JP)**
Inventor: **YABUTA, Tomoko**
**Asahigaokashataku K-902**
**161-55, Tsunoshita**
**Daimon-cho**
**Fukuyama-shi**
**Hiroshima 721 (JP)**

(74) Representative: **VOSSIUS & PARTNER**
**Siebertstrasse 4**
**D-81675 München (DE)**

(54) **NOVEL PYRIMIDINE DERIVATIVE AND MEDICINAL COMPOSITION.**

(57) A pyrimidine derivative represented by general formula (I) or a salt thereof, having an angiotensin II antagonism. In said formula, A represents (I-1) or (I-2), $R^1$ and $R^2$ represent each independently hydrogen, alkyl, alkenyl, alkynyl, alkoxy, alkylthio, acetyl, halogen, phenyl or substituted phenyl; $R^3$ represents hydrogen, alkyl, alkenyl, alkynyl or acetyl; X represents O, MH or S(O)p; p represents an integer of 0 to 2; m represents an integer of 1 or 2; B represents carboxy, alkoxycarbonyl, cyano, tetrazolyl or protected tetrazolyl; and n represents an integer of 1 to 2.



Rank Xerox (UK) Business Services
(3.10/3.09/3.3.4)

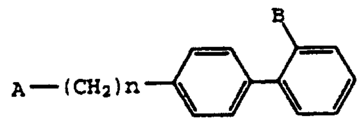

(I)

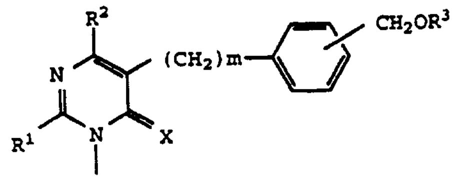

(I-1)

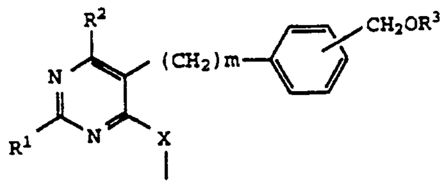

(I-2)

## TECHNICAL FIELD

The present invention relates to a novel pyrimidine derivative, and a pharmaceutical composition, more particularly, an angiotensin II antagonist, containing the pyrimidine derivative as an effective ingredient. The above-mentioned novel pyrimidine derivative according to the present invention exhibits an angiotensin II antagonism, and so is useful for various cardiovascular diseases, for example, hypertension, cardiac disease, cerebral infarction, and arteriosclerosis.

## BACKGROUND ART

Renin-angiotensin system is known as a major factor to maintain blood pressure. It is believed that the active renin (i.e., proteinase) secreted from kidney acts characteristically on the renin substrate (angiotensinogen) produced in liver to produce angiotensin I (AI), then, the resulting AI is converted to angiotensin II (AII) by the angiotensin converting enzyme (ACE) produced in lung, and the resulting AII exhibits a strong activity to raise blood pressure. Therefore, an AII antagonist may be used for the treatment of cardiovascular diseases, such as hypertension.

Hitherto, many angiotensin II analogues, such as [$Sar^1$, $Ala^8$] AII (saralasin), was reported to exhibit an angiotensin II antagonism. However, these AII antagonists had disadvantages in that the duration to exhibit efficacy is short and no effect is exhibited in oral administration. Further, imidazole derivatives as non-peptide type AII antagonists were disclosed in many patent publications; for example, Japanese Unexamined Patent Publications (Kokai) No. 56-71074, No. 57-98270, No. 58-157768, No. 63-23868, and No. 1-287071. Further, pyrimidine derivatives have been disclosed in European Publication No. 0407342, Japanese Unexamined Patent Publications (Kokai) No. 3-133964, No. 3-197466, No. 4-120072, No. 4-230370, No. 4-261156, and No. 4-330073, and European Publication No. 0445811.

## DISCLOSURE OF INVENTION

The inventors of the present invention engaged in intensive research to obtain an agent for the cardiovascular system which exhibits a superior AII antagonism, can be administered orally, and is low toxicity, and as a result, found that a group of pyrimidine derivatives having a chemical structure different from that of the conventional pyrimidine derivatives and carrying a substituted phenylalkyl group at 5-position of the pyrimidine ring exhibits a strong antagonism. The present invention is based on this finding.

Accordingly, the present invention relates to a pyrimidine derivative of the general formula (I):

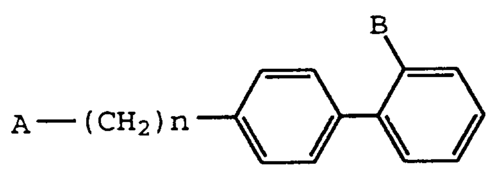

(I)

wherein A is a group of the general formula (I-1):

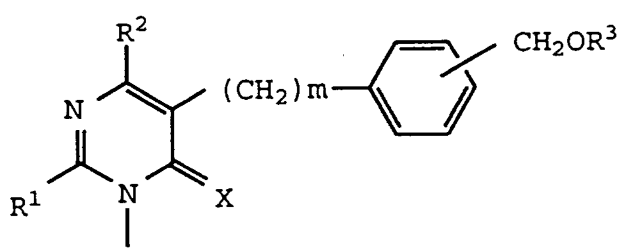

(I-1)

wherein $R^1$ and $R^2$, which may be same or different, are a hydrogen or hologen atom, lower alkyl, lower alkenyl, lower alkynyl, lower alkoxy, lower alkylthio, acetyl, phenyl, or substituted phenyl group, $R^3$ is a hydrogen atom, lower alkyl, lower alkenyl, lower alkynyl, or acetyl group, X is O, NH, or $S(O)_p$, p is an integer of 0 to 2, and m is an integer of 1 to 2, or a group of the general formula (I-2):

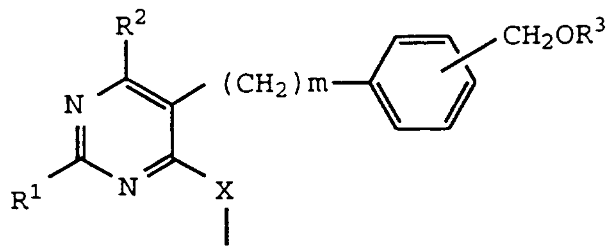

(I-2)

wherein $R^1$, $R^2$, $R^3$, X, p, and m have the same meanings as above, B is a carboxyl, lower alkoxycarbonyl, cyano, tetrazolyl, or protected tetrazolyl group, and n is an integer of 1 to 2, or a salt thereof.

Further, the present invention relates to a pharmaceutical composition, in particular an angiotensin II antagonist, characterized by containing the pyrimidine derivative of the general formula (I) or pharmaceutically acceptable salt thereof as an effective ingredient.

BEST MODE FOR CARRYING OUT THE INVENTION

The term "lower alkyl group" used herein means a straight or branched alkyl group with 1 to 6 (in particular 1 to 4) carbon atoms, such as, methyl, ethyl, propyl, isopropyl, n-butyl, isobutyl, sec-butyl, t-butyl, n-pentyl, isopentyl, neopentyl, t-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, or 1-ethyl-2-methylpropyl group, or the like.

The term "lower alkenyl group" means a straight or branched alkenyl group with 2 to 6 (in particular 2 to 4) carbon atoms, such as, vinyl, allyl, 1-propenyl, isopropenyl, 1-butenyl, 2-butenyl, 3-butenyl, 1-methyl-1-propenyl, 2-methyl-1-propenyl, 1-methylallyl, 2-methylallyl, 1-pentenyl, 2-pentenyl, 3-pentenyl, 4-pentenyl, 1-methyl-1-butenyl, 1-methyl-2-butenyl, 1-methyl-3-butenyl, 2-methyl-1-butenyl, 2-methyl-2-butenyl, 2-methyl-3-butenyl, 3-methyl-1-butenyl, 3-methyl-2-butenyl, 3-methyl-3-butenyl, 1,1-dimethylallyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, or 5-hexenyl group, or the like.

The lower alkynyl group may be a straight or branched alkynyl group with 2 to 6 (in particular 2 to 4) carbon atoms, such as, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-hexynyl, 2-hexynyl, 3-hexynyl, 4-hexynyl, or 5-hexynyl group, or the like.

The lower alkoxy group may be a straight or branched alkoxy group with 1 to 6 (in particular 1 to 4) carbon atoms, such as, methoxy, ethoxy, n-propoxy, isopropoxy, n-butoxy, isobutoxy, t-butoxy, n-pentyloxy, or n-hexanoxy group, or the like.

The lower alkylthio group may be a straight or branched alkylthio group with 1 to 6 (in particular 1 to 4) carbon atoms, such as, methylthio, ethylthio, n-propylthio, isopropylthio, n-butylthio, isobutylthio, sec-butylthio, t-butylthio, n-pentylthio, or n-hexylthio group, or the like.

The halogen atom is, for example, fluorine, chlorine, bromine, or iodine atom.

Examples of the substituent of the substituted phenyl group are a lower alkyl group with 1 to 6 (in particular 1 to 4) carbon atoms, a lower alkoxy group with 1 to 6 (in particular 1 to 4) carbon atoms, a halogen atom, nitro, trifluoromethyl, carboxyl, or lower alkoxycarbonyl group, or the like.

The lower alkoxycarbonyl group may be an alkoxycarbonyl group with 2 to 6 (in particular, 2 to 4) carbon atoms, such as, methoxycarbonyl, ethoxycarbonyl, n-propoxycarbonyl, isopropoxycarbonyl, n-butoxycarbonyl, isobutoxycarbonyl, sec-butoxycarbonyl, t-butoxycarbonyl, or n-pentyloxycarbonyl group, or the like.

The protecting group for the tetrazolyl group may be trityl, p-nitrobenzyl, 1-ethoxyethyl or t-butoxycarbonyl group, or the like.

As a preferred compound of the general formula (I), there may be mentioned the compound wherein $R^1$ is a lower alkyl group of 1 to 6 carbon atoms, $R^2$ is a lower alkyl group of 1 to 4 carbon atoms, $R^3$ is a hydrogen atom, a lower alkyl group of 1 to 2 carbon atoms, or a lower alkanoyl group of 2 to 4 carbon atoms, X is O, m is 1, B is a cyano, tetrazolyl, or a protected tetrazolyl group, and n is 1.

Further, as a more preferred compound of the general formula (I), there may be mentioned the compound wherein $R^1$ is a methyl, ethyl, propyl, butyl, or pentyl group, $R^2$ is a methyl, ethyl, or propyl

group, $R^3$ is a hydrogen atom, methyl, or acetyl group, X is O, m is 1, B is a cyano, tetrazolyl, or trityl tetrazolyl group, and n is 1.

As the pharmaceutically acceptable salt of the compound (I) of the present invention, there may be mentioned, alkali metal salts, such as sodium or potassium salts, alkali earth metal salts, such as magnesium or calcium salts, organic amine salts, such as methylamine, ethylamine, triethylamine, or pyridine salts, basic amino acid salts, such as lysine or arginine salts, or ammonium salts, or the like.

The compound (I) of the present invention may be one of geometrical isomers, optical isomers, or tautomers. One of the above isomers or the mixtures thereof is involved in the scope of the present invention.

The compound (I) of the present invention may be prepared, for example, by the following method. That is, a compound of the general formula (IV):

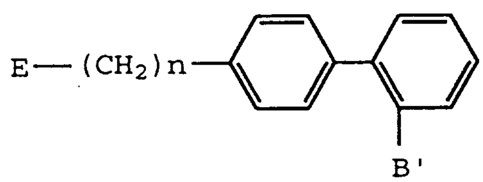

(IV)

wherein E is a group of the general formula (IV-1):

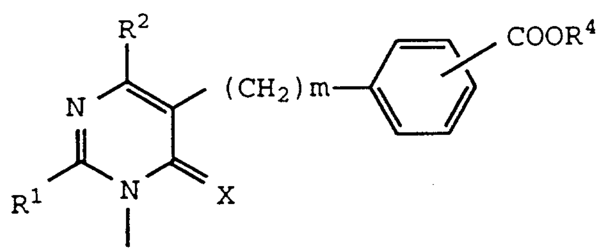

(IV-1)

wherein $R^1$, $R^2$, X and m have the same meanings as the above, and $R^4$ is a hydrogen atom, lower alkyl, or substituted lower alkyl group, or a group of the general formula (IV-2):

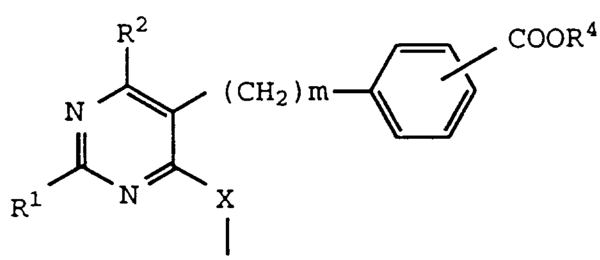

(IV-2)

wherein $R^1$, $R^2$, $R^4$, X and m have the same meanings as the above, B' is a lower alkoxycarbonyl, cyano, or protected tetrazolyl group, and n has the same meaning as the above, may be prepared by reacting, in the presence of a base, a compound of the general formula (II):

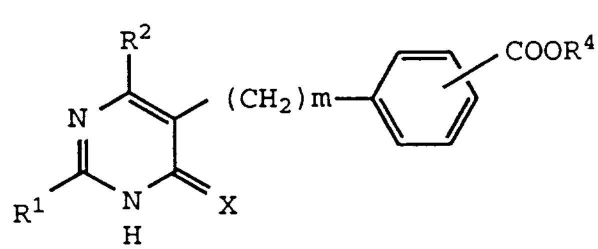

(II)

wherein $R^1$, $R^2$, $R^4$, X, and m have the same meanings as the above, and a compound of the general formula (III):

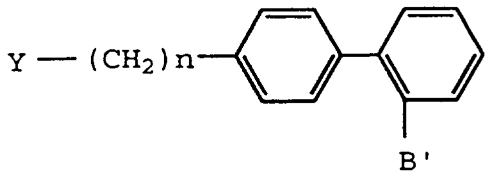

(III)

wherein B' and n have the same meanings as the above, and Y is a halogen atom, alkylsulfonyloxy, or arylsulfonyloxy group.

As examples of the base which can be used in the above reaction, there may be mentioned alkali metal compounds, such as sodium hydroxide, potassium hydroxide, sodium hydride, sodium methoxide, sodium ethoxide, or potassium t-butoxide; alkali metal carbonates, such as sodium carbonate, potassium carbonate, sodium hydrogencarbonate, or potassium hydrogencarbonate; or organic amines, such as triethylamine, diisopropylethylamine, pyridine, or N,N-dimethylaminopyridine. Solvents which do not take part in the reaction may be used. Examples of the solvent are an organic solvent, such as N,N-dimethylformamide, dimethylsulfoxide, N-methylpyrrolidone, dioxane, tetrahydrofuran, acetone, or alcohol, or a mixtures of one or more of the above solvents with water. The reaction temperature is in the range of -20 to 200 °C, preferably 0 to 100 °C, and the reaction is generally completed in 1 to 48 hours.

From the compound of the general formula (IV), the compound of the present invention of the general formula (Ia):

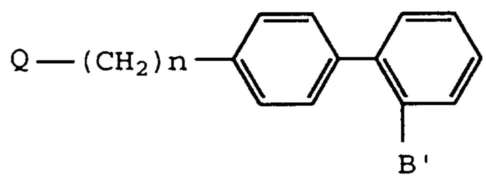

(Ia)

wherein Q is a group of the general formula (Ia-1):

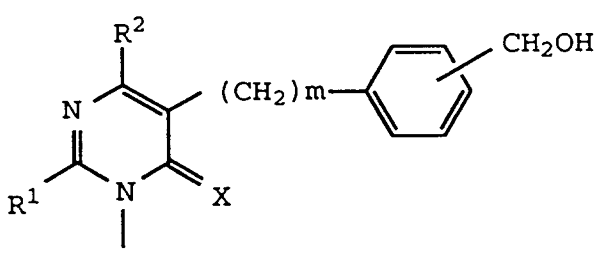

(Ia-1)

wherein $R^1$, $R^2$, X, and m have the same meanings as the above, or a group of the general formula (Ia-2):

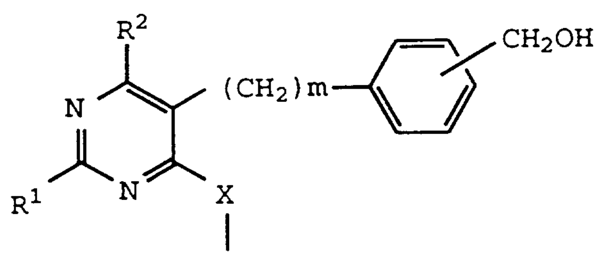

(Ia-2)

wherein $R^1$, $R^2$, X and m have the same meanings as the above, and B' and n have the same meanings as the above may be prepared in accordance with known methods, for example, the methods described in K. Soai et al., Bull. Chem. Soc. Jpn., 57, 1948 (1984), or G. Kototos, Syntehsis, 299 (1990).

As the reducing agent which may be used in the above reaction, there may be mentioned lithium aluminum hydride, or sodium borohydride, or a homologue thereof. The reducing agent may be optionally used in the presence of an appropriate Lewis acid, such as aluminum chloride, or cesium chloride. Solvents which do not take part in the reaction may be used. Examples of the solvent are tetrahydrofuran, diethyl ether, dioxane, methanol, ethanol, or dichloromethane. The reaction temperature is in the range of -20 to 100 ° C, preferably -10 to 60 ° C, and the reaction is generally completed in 30 minutes to 5 hours.

The compound of the general formula (Ia) of the present invention may be alkylated with an alkylating agent in the presence of a base to obtain a compound of the present invention of the general formula (Ib):

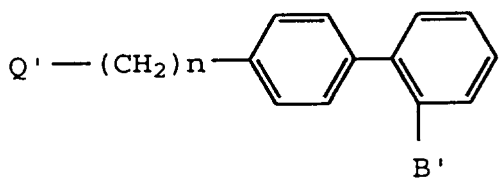

(Ib)

wherein Q' is a group of the general formula (Ib-1):

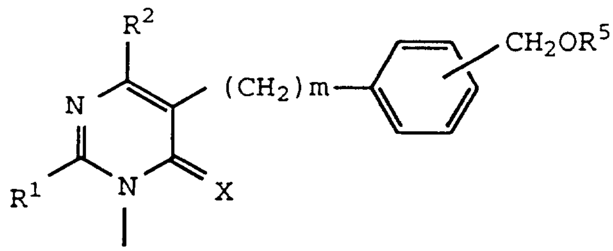

(Ib-1)

wherein $R^1$, $R^2$, X, and m have the same meanings as the above, and $R^5$ is a lower alkyl group, or a group of the general formula (Ib-2):

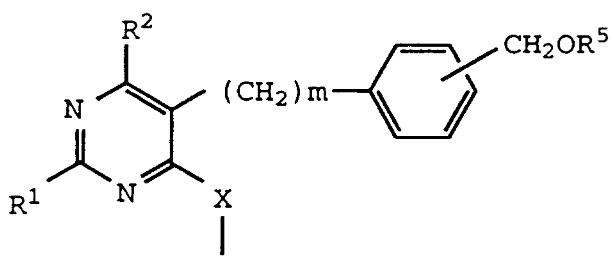

(Ib-2)

wherein $R^1$, $R^2$, $R^5$, X, and m have the same meanings as the above, and B' and n have the same meanings as the above.

The alkylation reaction may be generally performed, using approximately 1 to 3 moles of the base and approximately 1 to 3 moles of the alkylating agent with respect to 1 mole of the compound (I) of the present invention, in solvent, such as N,N-dimethylformamide, dimethylsulfoxide, acetonitrile, acetone, or ethyl methyl ketone. As the base which may be used in the alkylation reaction, there may be mentioned, for example, sodium hydroxide, potassium t-butoxide, potassium carbonate, or sodium carbonate. As the alkylating agent, there may be mentioned a substituted halide, such as chloride, bromide, or iodide, or substituted sulfonic acid esters, such as methyl p-toluenesulfonate. The reaction conditions vary with a combination of the base and the alkylating agent used, but the preferred alkylation reaction is performed generally at the ice-cold temperature to room temperature for approximately 1 to 48 hours.

The compound of the general formula (Ib) of the present invention wherein Q' is the group of the general formula (Ib-1) or (Ib-2) wherein $R^5$ is a lower alkenyl, lower alkynyl, or acetyl group, may be

prepared by reacting, as in the above alkylation, the compound of the general formula (Ia) of the present invention with a corresponding alkenylating agent, alkynylating agent or acetylating agent.

The compound of the general formula (I) of the present invention wherein B is a tetrazolyl group may be prepared by removing a protecting group from the correspounding compound of the general formula (I) of the present invention wherein B is a pretecting tetrazolyl group. Because of simplicity, the reaction is preferably performed in water-containing alcohol, such as methanol, or ethanol, or the like, containing approximately 0.5 to 2N hydrochloric or acetic acid, or in trifluoroacetic acid at ordinary temperature to 50 °C for approximately 1 to 10 hours.

Further, the compound of the general formula (I) of the present invention wherein B is the tetrazolyl group may be also prepared by reacting a correspounding compound of the general formula (I) of the present invention wherein B is a nitrile group with an azide derivative. As the azide derivative used in the reaction, there may be mentioned sodium azide, potassium azide, trimethyltin azide, or trimethyltin azide. The reaction may be performed in the presence or absence of a solvent which does not take part in the reaction, such as benzene, toluene, or xylene, at 0 to 200 °C for 30 minutes to 1 week to prepare the compound.

Further, the compound of the general formula (IV), (Ia) or (Ib) wherein B' is the tetrazolyl group may be also prepared from a compound having a corresponding nitrile group, in the similar manner as above.

The compound of the general formula (I) of the present invention wherein B is a carboxyl group may be also prepared by hydrolizing a corresponding compound of the general formula (I) of the present invention wherein B is a lower alkoxycarbonyl group in the presence of an acid or a base. The acid which may be used in the reaction is, for example, hydrochloric, or sulfuric acid, or the like, and the base which may be used is, for example, sodium hydroxide, or potassium hydroxide, or the like. The solvent is preferably a mixture of water and an alcohol, such as methanol, or ethanol, or the like. In general, the reaction is completed in the temperature range of ordinary temperature to reflux in 10 minutes to 10 hours.

The compound of the general formula (I) of the present invention wherein B is a lower alkoxycarbonyl group may be also prepared by esterifying a corresponding compound of the general formula (I) of the present invention wherein B is a carboxyl group in the presence of an acid. The acid which may be used in the reaction is, for example, hydrochloric, sulfuric, or arylsulfonic acid, or the like. The solvent which may be used in the reaction is an alcohol which is also a reactant, such as methanol, ethanol, propanol, or butanol, or the like. The reaction is completed in the temperature range of ordinary temperature to reflux in 1 to 24 hours.

A compound of the present invention of the general formula (Ic):

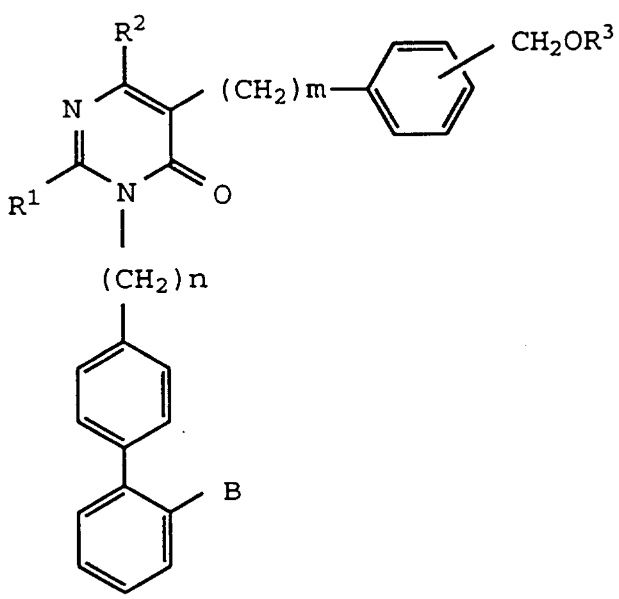

(Ic)

wherein $R^1$, $R^2$, $R^3$, B, m, and n have the same meanings as the above, may be prepared by reacting a compound of the general formula (V):

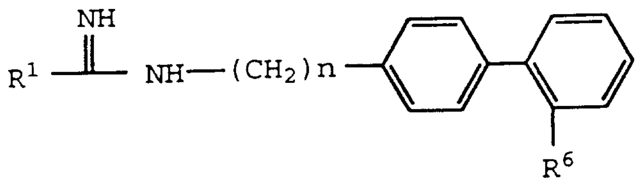

(V)

wherein $R^1$ and n have the same meanings as above, and $R^6$ is a nitrile or protected tetrazolyl group, with a compound of the general formula (VI):

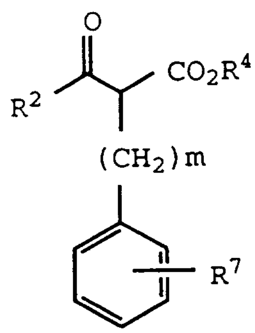

(VI)

wherein $R^2$, $R^4$, and m have the same meanings as above, and $R^7$ is a lower alkoxycarbonyl, hydroxymethyl, or lower alkoxymethyl group, to form a pyrimidine ring, and then, converting $R^6$ and $R^7$ in the similar manner as above.

Further, a compound of the present invention of the general formula (Id):

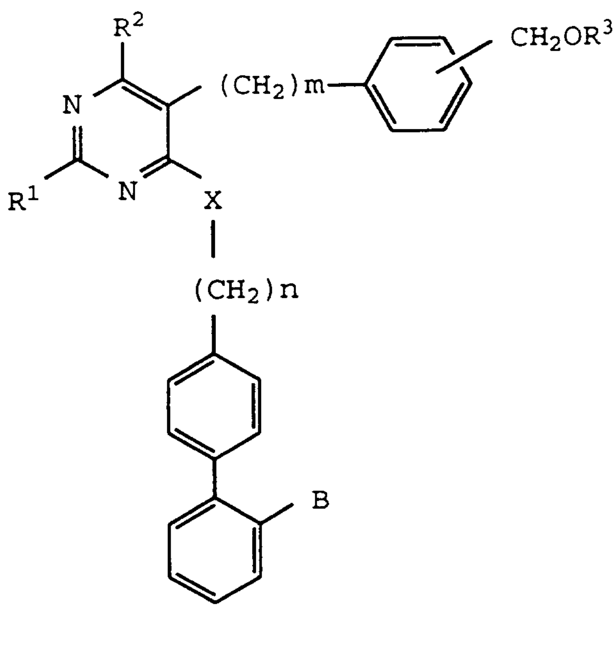

(Id)

wherein $R^1$, $R^2$, $R^3$, B, m, and n have the same meanings as the above, is prepared by reacting a compound of the general formula (VII):

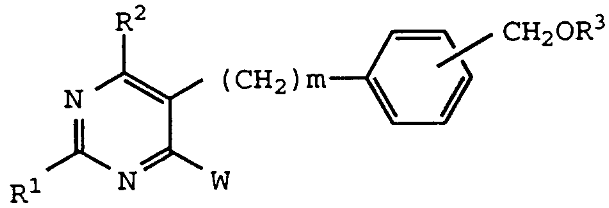

(VII)

wherein $R^1$, $R^2$, $R^3$, and m have the same meanings as above, and W is a halogen atom or nitro group, with a compound of the general formula (VIII):

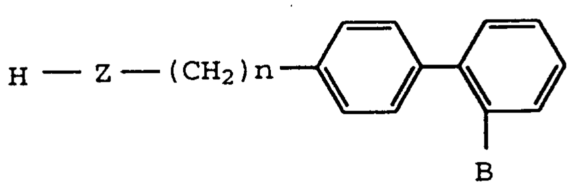

(VIII)

wherein B and n have the same meanings as above, and Z is O, NH or S, under the similar reaction conditions to those for preparation of the compound of the general formula (IV), and then, optionally converting $R^3$ and/or B into other $R^3$ and/or B.

The starting compounds used for the aboved-mentioned processes are known per se or may be prepared by known methods. For example, the pyrimidine derivative of the general formula (II) may be prepared in accordance with the method described in Japanese Unexamined Patent Publication (Kokai) No. 3-133964. Further, the biphenyl derivatives of the general formulae (III) and (VIII) may be prepared in accordance with the method described in WO-89/06233 or Japanese Unexamined Patent Publication (Kokai) No. 1-117876.

The compound of the general formula (I) of the present invention prepared by the above-mentioned processes may be purified by ordinary methods for purification, such as recrystallization, reprecipitation, solvent extraction, silica gel column chromatography, or column chromatography with adsorptive resin, or the like.

The compound of the general formula (I) of the present invention has an angiotensin II antagonism and is low toxicity, and thus may be used as a pharmaceutical composition, particularly, an angiotensin II antagonist, for the treatment of various cardiovascular diseases. Example of such cardiovascular diseases are hypertension, cardiac disease, cerebral infarction, arteriosclerosis, or the like. Further, the compound of the general formula (I) of the present invention is effective as an agent for the treatment of high intraocular pressure.

A pharmaceutical composition, in particular, an angiotensin II antagonist, containing the compound of the general formula (I) of the present invention or a pharmaceutically acceptable salt thereof as an effective ingredient may be administered orally or parenterally, for example, intramuscularly, intravenously, subcutaneously, percutaneously, or per rectum, or the like. The composition may be formulated with one or more pharmaceutically acceptable auxiliaries to form tablets, capsules, granules, dispersions, pills, fine granules, injections, rectal agents, suppositories, or the like. As the pharmaceutically acceptable auxiliaries, there may be mentioned excipients, binding agents, disintegrating agents, lubricants, buffers, preservatives, solubilizing auxiliaries, antiseptics, agents for adjusting taste and/or smell, analgesics, stabilizers, coloring agents, or the like. The auxiliaries as above may be appropriately combined by ordinary methods.

As the excipients, lactose, sucrose, glucose, sorbitol, corn starch, crystalline cellulose, or the like may be used. As the binding agent, a cellulose derivative, gum arabic, gelatine, polyvinyl alcohol, polyvinyl ether, or the like may be used. The disintegrating agent is, for example, calcium carboxylmethylcellulose or the like. The lubricant is, for example, talc, magnesium stearate, polyethyleneglycol or the like. The preservative is, for example, methyl paraoxybenzoate, ethyl paraoxybenzoate, sorbic acid, phenol, cresol, chlorocresol or the like. The solubilizing auxiliary is, for example, polyoxyethylene hardened castor oil, polysorbate 80, nicotinic acid amide, polyoxyethylene sorbitan monolaurate, macrogol, or the like. The agent for adjusting taste and/or smell is, for example, cocoa powder, peppermint oil, cinnamin powder, or the like. The stabilizer is, for example, sodium sulfite, sodium metasulfite, ether, or the like.

A pharmaceutical composition, in particular, an angiotensin II antagonist, of the present invention, contains the compound of the general formula (I) of the present invention or a pharmaceutically acceptable salt thereof as an effective ingredient in an amount effective for an angiotensin II antagonism, for example, 5 to 100 % by weight, preferably 25 to 100 % by weight. The dose effective for the angiotensin II antagonism varies with the age, body weight, sex, symptoms, method of administration, period of administration, desired therapeutic effect, or the like, but in the case of oral administration, the dose is normally 1 to 1000 mg, preferably 5 to 200 mg, per day for an adult, usually divided into one to three dosages.

Examples

The present invention now will be further illustrated by, but is by no means limited to, the following Reference Examples and Examples.

Reference Example 1: Preparation of 5-(4-methoxycarbonylbenzyl)-6-methyl-2-propylpyrimidin-4(3H)-one

Sodium methoxide (2.70 g) was dissolved in anhydrous methanol (60 ml). To the solution, n-propylamidine hydrochloride (6.13 g) was added. The whole was stirred at room temperature for 10 minutes. Then, ethyl 2-(4-methoxycarbonylbenzyl)-3-oxobutyrate (6.13 g) and absolute methanol (60 ml) were added, and the mixture was stirred at 50 °C overnight. After the solvent was evaporated under reduced pressure, water was added. The mixture was rendered to weak acidic with 1N hydrochloric acid, and extracted with ethyl acetate twice. The ethyl acetate extract layer was washed with water and saturated saline, and dried over anhydrous sodium sulfate. The solvent was evaporated under reduced pressure, and the resulting crystals were washed with n-hexane to obtain the above-titled compound (4.69 g).

Yield: 63%,

Melting point: 210 °C (decomposition)

$^1$H-NMR ($CDCl_3$)δ: 0.96 (3H, t, J = 7.4Hz), 1.70-1.85 (2H, m), 2.34 (3H, s), 2.56-2.63 (2H, m), 3.89 (3H, s), 3.94 (2H, s), 7.32 (2H, d, J = 8.6Hz), 7.93 (2H, d, J = 8.6Hz)

IR (KBr) $cm^{-1}$: 1725, 1660, 1605, 1275

MS (FAB) m/z: 301 (M + 1)

Reference Examples 2 to 20

The compounds of the following Reference Examples 2 to 42 were prepared in accordance with the method described in Reference Example 1.

Reference Example 2: 5-(4-methoxycarbonylbenzyl)-2,6-dimethylpyrimidin-4(3H)-one

Yield: 65%,

Melting point: 224-225 °C

Reference Example 3: 2,6-diethyl-5-(4-methoxycarbonylbenzyl)pyrimidin-4(3H)-one

Yield: 78%,

Melting point: 177-179 °C

Reference Example 4: 6-ethyl-5-(4-methoxycarbonylbenzyl)-2-propylpyrimidin-4(3H)-one

Yield: 72%,

Melting point: 151-152 °C

Reference Example 5: 2-butyl-6-ethyl-(4-methoxycarbonylbenzyl)pyrimidin-4(3H)-one

Yield: 76%,

Melting point: 140-141 °C

Reference Example 6: 5-(4-methoxycarbonylbenzyl)-6-methyl-2-pentylpyrimidin-4(3H)-one

Yield: 85%,
Melting point: 178-179 ° C

Reference Example 7: 5-(3-methoxycarbonylbenzyl)-2,6-dimethylpyrimidin-4(3H)-one

Yield: 42%,
Melting point: 173-175 ° C

Reference Example 8: 2-ethyl-5-(3-methoxycarbonylbenzyl)-6-methylpyrimidin-4(3H)-one

Yield: 50%,
Melting point: 180-181 ° C

Reference Example 9: 5-(3-methoxycarbonylbenzyl)-6-methyl-2-propylpyrimidin-4(3H)-one

Yield: 65%,
Melting point: 161-162 ° C

Reference Example 10: 2-butyl-5-(3-methoxycarbonylbenzyl)-6-methylpyrimidin-4(3H)-one

Yield: 80%,
Melting point: 171-173 ° C

Reference Example 11: 5-(3-methoxycarbonylbenzyl)-6-methyl-2-pentylpyrimidin-4(3H)-one

Yield: 65%,
Melting point: 155-156 ° C

Reference Example 12: 6-ethyl-5-(3-methoxycarbonylbenzyl)-2-methylpyrimidin-4(3H)-one

Yield: 77%,
Melting point: 138-140 ° C

Reference Example 13: 2,6-diethyl-5-(3-methoxycarbonylbenzyl)pyrimidin-4(3H)-one

Yield: 70%,
Melting point: 133-135 ° C

Reference Example 14: 6-ethyl-5-(3-methoxycarbonylbenzyl)-2-propylpyrimidin-4(3H)-one

Yield: 71%,
Melting point: 111-113 ° C

Reference Example 15: 2-butyl-6-ethyl-(3-methoxycarbonylbenzyl)pyrimidin-4(3H)-one

Yield: 50%,
Melting point: 93-95 ° C

Reference Example 16: 5-(3-methoxycarbonylbenzyl)-2-methyl-6-propylpyrimidin-4(3H)-one

Yield: 62%,
Melting point: 131-133 ° C

Reference Example 17: 2-ethyl-5-(3-methoxycarbonylbenzyl)-6-propylpyrimidin-4(3H)-one

Yield: 56%,
Melting point: 122-124 ° C

Reference Example 18: 5-(3-methoxycarbonylbenzyl)-2,6-dipropylpyrimidin-4(3H)-one

Yield: 53%,
Melting point: 109-110 ° C

Reference Example 19: 2-butyl-5-(3-methoxycarbonylbenzyl)-6-propylpyrimidin-4(3H)-one

Yield: 61%,
Melting point: 115-117 ° C

Reference Example 20: 6-ethyl-5-(4-methoxycarbonylbenzyl)-2-methylpyrimidin-4(3H)-one

Yield: 61%,
Melting point: 186-187 ° C

Reference Example 21: Preparation of 5-(4-carboxybenzyl)-6-methyl-2-propyl-4-[2'-(1H-tetrazol-5-yl)-biphenyl-4-yl]methoxypyrimidine

(a) Preparation of 5-(4-methoxycarbonylbenzyl)-6-methyl-2-propyl-4-[2'-(triphenylmethyltetrazol-5-yl)-biphenyl-4-yl]methoxypyrimidine

Sodium methoxide (0.65 g) was suspended in dry N,N-dimethylformamide (40 ml). The suspension was stirred at room temperature for 20 minutes. Then, 5-(4-methoxycarbonylbenzyl)-6-methyl-2-propylpyrimidin-4(3H)-one (3.60 g) and dry N,N-dimethylformamide (40 ml) were added and the mixture was stirred further for 1 hour. Then, 4'-bromomethyl-2-(triphenylmethyltetrazol-5-yl)biphenyl (7.35 g) was dissolved in dry N,N-dimethylformamide (40 ml) and the resulting solution was added dropwise to the reaction mixture. The whole was stirred at room temperature for 60 hours. After the reaction was completed, the reaction mixture was concentrated and water (40 ml) was added. Then, extraction was performed twice with ethyl acetate (150 ml). The ethyl acetate extract layer was washed with water and saturated saline, and then, dried over anhydrous sodium sulfate. The solvent was evaporated under reduced pressure and the resulting residue was purified, by silica gel column chromatography, eluting with n-hexane/ethyl acetate (3:1) to obtain the above-titled compound (2.92 g) as amorphous.

Yield: 31%

$^1$H-NMR ($CDCl_3$)$\delta$: 0.90 (3H, t, J = 7.2Hz), 1.73-1.85 (2H, m), 2.38 (3H, s), 2.80 (2H, t, J = 7.8Hz), 3.86 (3H, s), 3.98 (2H, s), 5.34 (2H, s), 6.88-6.96 (8H, m), 7.12 (2H, d, J = 8.3Hz), 7.21-7.36 (12H, m), 7.43-7.51 (2H, m), 7.92-7.97 (3H, m)

MS (FAB) m/z: 777 (M + 1)

(b) Preparation of 5-(4-carboxybenzyl)-6-methyl-2-propyl-4-[2'-(1H-tetrazol-5-yl)biphenyl-4-yl]-methoxypyrimidine

The compound (1.10 g) prepared in Reference Example 21 (a) was dissolved in methanol (50 ml), and several drops of concentrated hydrochloric acid were added. The mixture was stirred at room temperature for 4 hours. Then, 2N NaOH (4.0 ml) was added and the whole was heated under reflux for 7 hours. After the reaction was completed, the reaction mixture was concentrated to a solid under reduced pressure. Water (30 ml) was added to dissolve the resulting residue. The insolubles were filtered out. The pH of the resulting solution was adjusted to 4 with 1N hydrochloric acid. The precipitated crystals were collected by filtration to obtain the above-titled compound (0.67 g) as white powder.

Yield: 91%,
Melting point: 167-169 ° C

$^1$H-NMR (DMSO-$d_6$)$\delta$: 0.92 (3H, t, J = 7.5Hz), 1.69-1.82 (2H, m), 2.36(3H, s), 2.70 (2H, t, J = 7.4Hz), 4.01 (2H, s), 5.42 (2H, s), 7.06 (2H, d, J = 8.3Hz), 7.21 (2H, d, J = 8.1Hz), 7.24 (2H, d, J = 8.3Hz), 7.49-7.68 (4H, m), 7.82(2H, d, J = 8.3Hz)

IR (KBr) $cm^{-1}$: 1610, 1570, 1425, 1345
MS (FAB) m/z: 521 (M + 1)

Reference Example 22: Preparation of 5-(4-carboxybenzyl)-6-methyl-2-propyl-3-[2'-(1H-tetrazol-5-yl)-biphenyl-4-yl]methylpyrimidin-4-one

(a) Preparation of 5-(4-methoxycarbonylbenzyl)-6-methyl-2-propyl-3-[2'-(triphenylmethyltetrazol-5-yl)-biphenyl-4-yl]methylpyrimidin-4-one

The above-titled compound (3.25 g) was obtained from the n-hexane/ethyl acetate (2:1) eluate in the purification step of the reaction product of Reference Example 21 (a) by silica gel column chromatography.
Yield: 35%
$^1$H-NMR ($CDCl_3$)δ: 0.87 (3H, t, J = 7.2Hz), 1.58-1.66 (2H, m), 2.29 (3H, s), 2.53 (2H, t, J = 7.8Hz), 3.89 (3H, s), 3.99 (2H, s), 5.22 (2H, s), 6.87-6.90 (6H, m), 7.01-7.30 (15H, m), 7.35-7.54 (3H, m), 7.86-7.96 (3H, m)
MS (FAB) m/z: 777 (M + 1)

(b) Preparation of 5-(4-carboxybenzyl)-6-methyl-2-propyl-3-[2'-(1H-tetrazol-5-yl)biphenyl-4-yl]-methylpyrimidin-4-one

The compound (3.25 g) prepared in Reference Example 22 (a) was treated in accordance with the method described in Reference Example 21 (b) to obtain the above-titled compound (2.04 g) as white powder.
Yield: 94%,
Melting point: 145-146 ° C
$^1$H-NMR (DMSO-$d_6$)δ: 0.86 (3H, t, J = 7.3Hz), 1.54-1.68 (2H, m), 2.23(3H, s), 2.60 (2H, t, J = 7.4Hz), 3.92 (2H, s), 5.30 (2H, s), 7.08 (4H, s), 7.31 (2H, d, J = 8.1Hz), 7.49-7.57(2H, m), 7.62-7.68(2H, m), 7.84 (2H, d, J = 8.3Hz)
IR (KBr) $cm^{-1}$: 1705, 1650, 1610, 1540
MS (FAB) m/z: 521 (M + 1)

Reference Examples 23-57

The compounds of the following Reference Examples 23 to 57 were prepared in accordance with the method described in Reference Examples 21 and 22.

Reference Example 23: 5-(4-carboxybenzyl)-2,6-dimethyl-3-[2'-(1H-tetrazol-5-yl)biphenyl-4-yl]-methylpyrimidin-4-one

Yield: 48%,
Melting point: 203-205 ° C
$^1$H-NMR (DMSO-$d_6$)δ: 2.20 (3H, s), 2.36 (3H, s), 3.91 (2H, s), 5.27 (2H, s), 7.09 (4H, s), 7.31 (2H, d, J = 8.3Hz), 7.50-7.58 (2H, m), 7.62-7.68 (2H, m), 7.84 (2H, d, J = 8.1Hz)
IR (KBr) $cm^{-1}$: 1655, 1540, 1180, 760
MS (FAB) m/z: 493 (M + 1)

Reference Example 24: 5-(4-carboxybenzyl)-2,6-diethyl-4-[2'-(1H-tetrazol-5-yl)biphenyl-4-yl]-methoxypyrimidine

Yield: 24%,
Melting point: 154-156 ° C
$^1$H-NMR (DMSO-$d_6$)δ: 1.10 (3H, t, J = 7.6Hz), 1.26 (3H, t, J = 7.6Hz), 2.68 (2H, q, J = 7.5Hz), 2.77 (2H, q, J = 7.6Hz), 4.03 (2H, s), 5.42 (2H, s), 7.05 (2H, d, J = 8.1Hz), 7.19-7.25 (4H, m), 7.50-7.69 (4H, m), 7.82 (2H, d, J = 8.3Hz)
IR (KBr) $cm^{-1}$: 1610, 1570, 1420, 1345
MS (FAB) m/z: 521 (M + 1)

Reference Example 25: 5-(4-carboxybenzyl)-2,6-diethyl-3-[2'-(1H-tetrazol-5-yl)biphenyl-4-yl]methylpyrimidin-4-one

Yield: 23%,

Melting point: 139-141 ° C

$^1$H-NMR (DMSO-$d_6$)δ: 1.08 (3H, t, J = 7.4Hz), 1.13 (3H, t, J = 7.2Hz), 2.54 (2H, q, J = 7.4Hz), 2.66 (2H, q, J = 7.3Hz), 3.95 (2H, s), 5.30 (2H, s), 7.09 (4H, s), 7.31 (2H, d, J = 8.3Hz), 7.50-7.68 (4H, m), 7.85 (2H, d, J = 8.3Hz)

IR (KBr) $cm^{-1}$: 1710, 1645, 1610, 1540

MS (FAB) m/z: 521 (M + 1)

Reference Example 26: 5-(4-carboxybenzyl)-6-ethyl-2-propyl-4-[2'-(1H-tetrazol-5-yl)biphenyl-4-yl]-methoxypyrimidine

Yield: 28%,

Melting point: 195 ° C (decomposition)

$^1$H-NMR (DMSO-$d_6$)δ: 0.93 (3H, t, J = 7.3Hz), 1.11 (3H, t, J = 7.4Hz), 1.73-1.81 (2H, m), 2.69 (2H, q, J = 7.4Hz), 2.75 (2H, t, J = 7.4Hz), 4.04 (2H, s), 5.43 (2H, s), 7.05 (2H, d, J = 8.3Hz), 7.19-7.24 (4H, m), 7.50-7.70 (4H, m), 7.82 (2H, d, J = 8.3Hz)

IR (KBr) $cm^{-1}$: 1610, 1570, 1420, 1345

MS (FAB) m/z: 535 (M + 1)

Reference Example 27: 5-(4-carboxybenzyl)-6-ethyl-2-propyl-3-[2'-(1H-tetrazol-5-yl)biphenyl-4-yl]-methylpyrimidin-4-one

Yield: 30%,

Melting point: 136-138 ° C

$^1$H-NMR (DMSO-$d_6$)δ: 0.87 (3H, t, J = 7.3Hz), 1.07 (3H, t, J = 7.4Hz), 1.59-1.68 (2H, m), 2.54(2H, q, J = 7.4Hz), 2.62 (2H, t, J = 7.3Hz), 3.94 (2H, s), 5.30 (2H, s), 7.09 (4H, s), 7.30 (2H, d, J = 8.3Hz), 7.50-7.69 (4H, m), 7.84 (2H, d, J = 8.3Hz)

IR (KBr) $cm^{-1}$: 3450, 2970, 1645, 1540

MS (FAB) m/z: 535 (M + 1)

Reference Example 28: 2-butyl-5-(4-carboxybenzyl)-6-ethyl-4-[2'-(1H-tetrazol-5-yl)biphenyl-4-yl]-methoxypyrimidine

Yield: 30%,

Melting point: 106-108 ° C

$^1$H-NMR (DMSO-$d_6$)δ: 0.90 (3H, t, J = 7.3Hz), 1.09 (3H, t, J = 7.4Hz), 1.28-1.41 (2H, m), 1.67-1.78 (2H, m), 2.66 (2H, q, J = 7.6Hz), 2.74 (2H, t, J = 7.6Hz), 4.02 (2H, s), 5.41 (2H, s), 7.05 (2H, d, J = 8.3Hz), 7.18-7.24 (4H, m), 7.50-7.69 (4H, m), 7.82 (2H, d, J = 8.3Hz)

IR (KBr) $cm^{-1}$: 1610, 1570, 1415, 1345

MS (FAB) m/z: 549 (M + 1)

Reference Example 29: 5-(4-carboxybenzyl)-6-methyl-2-pentyl-4-[2'-(1H-tetrazol-5-yl)biphenyl-4-yl]-methoxypyrimidine

Yield: 32%,

Melting point: 166-168 ° C

$^1$H-NMR (DMSO-$d_6$)δ: 0.86 (3H, t, J = 7.0Hz), 1.28-1.33 (4H,m), 1.68-1.78 (2H, m), 2.35 (3H, s), 2.71 (2H, t, J = 7.6Hz), 4.01 (2H, s), 5.41 (2H, s), 7.05 (2H, d, J = 8.1Hz), 7.19-7.26 (4H, m), 7.50-7.59 (2H, m), 7.63-7.69 (2H, m), 7.82 (2H, d, J = 8.1Hz)

IR (KBr) $cm^{-1}$: 1705, 1610, 1580, 1345

MS (FAB) m/z: 549 (M + 1)

Reference Example 30: 5-(4-carboxybenzyl)-6-methyl-2-pentyl-3-[2'-(1H-tetrazol-5-yl)biphenyl-4-yl]-methylpyrimidin-4-one

Yield: 34%,
Melting point: 140-141 ° C
$^1$H-NMR (DMSO-$d_6$)δ: 0.82 (3H, t, J=6.8Hz), 1.21-1.26 (4H, m), 1.55-1.60 (2H, m), 2.22 (3H, s), 2.60 (2H, t, J=7.6Hz), 3.92 (2H, s), 5.29 (2H, s), 7.08 (4H, s), 7.33 (2H, d, J=8.1Hz), 7.49-7.58 (2H, m), 7.63-7.69 (2H, m), 7.84 (2H, d, J=8.1Hz)
IR (KBr) $cm^{-1}$: 1705, 1660, 1610, 1535
MS (FAB) m/z: 549 (M+1)

Reference Example 31: 5-(3-carboxybenzyl)-2,6-dimethyl-4-[2'-(1H-tetrazol-5-yl)biphenyl-4-yl]-methoxypyrimidine

Yield: 8%,
Melting point: 151 ° C (decomposition)
$^1$H-NMR (DMSO-$d_6$)δ: 2.37 (3H, s), 2.48 (3H, s), 4.01 (2H, s), 5.41 (2H, s), 7.05 (2H, d, J=8.1Hz), 7.24 (2H, d, J=8.3Hz), 7.34-7.39 (2H, m), 7.50-7.78 (6H, m)
IR (KBr) $cm^{-1}$: 3440, 1575, 1425, 1345
MS (FAB) m/z: 493 (M+1)

Reference Example 32: 5-(3-carboxybenzyl)-2,6-dimethyl-3-[2'-(1H-tetrazol-5-yl)biphenyl-4-yl]-methylpyrimidin-4-one

Yield: 42%,
Melting point: 141 ° C (decomposition)
$^1$H-NMR (DMSO-$d_6$)δ: 2.21 (3H, s), 2.36 (3H, s), 3.91 (2H, s), 5.29 (2H, s), 7.10 (4H, s), 7.35-7.79 (8H, m)
IR (KBr) $cm^{-1}$: 3450, 1705, 1650, 1540
MS (FAB) m/z: 493 (M+1)

Reference Example 33: 5-(3-carboxybenzyl)-2-ethyl-6-methyl-4-[2'-(1H-tetrazol-5-yl)biphenyl-4-yl]-methoxypyrimidine

Yield: 26%,
Melting point: 84 ° C (decomposition)
$^1$H-NMR (DMSO-$d_6$)δ: 1.25 (3H, t, J=7.6Hz), 2.40 (3H, s), 2.76 (2H, q, J=7.6Hz), 4.02 (2H, s), 5.44 (2H, s), 7.06 (2H, d, J=8.3Hz), 7.26 (2H, d, J=8.3Hz), 7.34-7.36 (4H, m), 7.50-7.78(4H, m)
IR (KBr) $cm^{-1}$: 1605, 1575, 1420, 1345
MS (FAB) m/z: 507 (M+1)

Reference Example 34: 5-(3-carboxybenzyl)-2-ethyl-6-methyl-3-[2'-(1H-tetrazol-5-yl)biphenyl-4-yl]-methylpyrimidin-4-one

Yield: 33%,
Melting point: 93 ° C (decomposition)
$^1$H-NMR (DMSO-$d_6$)δ: 1.10 (3H, t, J=7.3Hz), 2.24 (3H, s), 2.64 (2H, q, J=7.2Hz), 3.92 (2H, s), 5.31 (2H, s), 7.08 (4H, s), 7.35-7.79 (8H, m)
IR (KBr) $cm^{-1}$: 3430, 1710, 1650, 1540
MS (FAB) m/z: 507 (M+1)

Reference Example 35: 5-(3-carboxybenzyl)-6-methyl-2-propyl-4-[2'-(1H-tetrazol-5-yl)biphenyl-4-yl]-methoxypyrimidine

Yield: 31%,
Melting point: 119-121 ° C
$^1$H-NMR (DMSO-$d_6$)δ: 0.91 (3H, t, J=7.3Hz), 1.68-1.79 (2H, m), 2.39 (3H, s), 2.71 (2H, t,J =7.4Hz), 4.02 (2H, s), 5.44 (2H, s), 7.06 (2H, d, J=8.3Hz), 7.25 (2H, d, J=8.3Hz), 7.34-7.37 (2H, m), 7.50-7.78 (6H, m)

IR (KBr) $cm^{-1}$: 1605, 1575, 1425, 1345
MS (FAB) m/z: 521 (M+1)

Reference Example 36: 5-(3-carboxybenzyl)-6-methyl-2-propyl-3-[2'-(1H-tetrazol-5-yl)biphenyl-4-yl]-methylpyrimidin-4-one

Yield: 34%,
Melting point: 128-130°C
$^1$H-NMR (DMSO-$d_6$)δ: 0.86 (3H, t, J=7.3Hz), 1.56-1.64 (2H, m), 2.23 (3H, s), 2.59 (2H, t, J=7.4Hz), 3.92 (2H, s), 5.31 (2H, s), 7.09 (4H, s), 7.35-7.80 (8H, m)
IR (KBr) $cm^{-1}$: 2970, 1705, 1650, 1535
MS (FAB) m/z: 521 (M+1)

Reference Example 37: 2-butyl-5-(3-carboxybenzyl)-6-methyl-4-[2'-(1H-tetrazol-5-yl)biphenyl-4-yl]-methoxypyrimidine

Yield: 23%,
Melting point: 115-117°C
$^1$H-NMR (DMSO-$d_6$)δ: 0.89 (3H, t, J=7.3Hz), 1.26-1.40 (2H, m), 1.58-1.77 (2H, m), 2.36 (3H, s), 2.72 (2H, t, J=7.6Hz), 4.01 (2H, s), 5.42 (2H, s), 7.06 (2H, d, J=8.1Hz), 7.24 (2H, d, J=8.1Hz), 7.33-7.44 (2H, m), 7.48-7.77 (6H, m)
IR (KBr) $cm^{-1}$: 1690, 1605, 1575, 1345
MS (FAB) m/z: 535 (M+1)

Reference Example 38: 2-butyl-5-(3-carboxybenzyl)-6-methyl-3-[2'-(1H-tetrazol-5-yl)biphenyl-4-yl]-methylpyrimidin-4-one

Yield: 32%,
Melting point: 128-130°C
$^1$H-NMR (DMSO-$d_6$)δ: 0.81 (3H, t, J=7.2Hz), 1.21-1.35 (2H, m), 1.51-1.62 (2H, m), 2.22 (3H, s), 2.61 (2H, t, J=7.6Hz), 3.92 (2H, s), 5.31 (2H, s), 7.09 (4H, s), 7.34-7.80 (8H, m)
IR (KBr) $cm^{-1}$: 2960, 1705, 1650, 1535
MS (FAB) m/z: 535 (M+1)

Reference Example 39: 5-(3-carboxybenzyl)-6-methyl-2-pentyl-4-[2'-(1H-tetrazol-5-yl)biphenyl-4-yl]-methoxypyrimidine

Yield: 28%,
Melting point: 161°C (decomposition)
$^1$H-NMR (DMSO-$d_6$)δ: 0.86 (3H, t, J=7.0Hz), 1.27-1.32 (4H, m), 1.70-1.75 (2H, m), 2.37 (3H, s), 2.71 (2H, t, J=7.6Hz), 4.01 (2H, s), 5.42 (2H, s), 7.04-7.07 (2H, m), 7.23-7.26 (2H, m), 7.34-7.36 (2H, m), 7.50-7.77 (6H, m)
IR (KBr) $cm^{-1}$: 3600-3300, 2950, 1710, 1575, 1345
MS (FAB) m/z: 549 (M+1)

Reference Example 40: 5-(3-carboxybenzyl)-6-methyl-2-pentyl-3-[2'-(1H-tetrazol-5-yl)biphenyl-4-yl]-methylpyrimidin-4-one

Yield: 31%,
Melting point: 136°C (decomposition)
$^1$H-NMR (DMSO-$d_6$)δ: 0.82 (3H, t, J=7.0Hz), 1.21-1.26 (4H, m), 1.55-1.61 (2H, m), 2.23 (3H, s), 2.60 (2H, t, J=7.7Hz), 3.92 (2H, s), 5.31 (2H, s), 7.09 (4H, s), 7.35-7.58 (4H, m), 7.64-7.69 (2H, m), 7.74-7.80 (2H, m)
IR (KBr) $cm^{-1}$: 3600-3300, 2960, 1715, 1655, 1445
MS (FAB) m/z: 549 (M+1)

Reference Example 41: 5-(3-carboxybenzyl)-6-ethyl-2-methyl-3-[2'-(1H-tetrazol-5-yl)biphenyl-4-yl]-methylpyrimidin-4-one

Yield: 31%,
Melting point: 143-145 °C
$^1$H-NMR (DMSO-$d_6$)δ: 1.05 (3H, t, J = 7.4Hz), 2.38 (3H, s), 2.52-2.55 (2H, m), 3.93 (2H, s), 5.29 (2H, s), 7.06-7.14 (4H, m), 7.35-7.77 (8H, m)
IR (KBr) $cm^{-1}$: 3440, 1705, 1655, 1540
MS (FAB) m/z: 507 (M + 1)

Reference Example 42: 5-(3-carboxybenzyl)-2,6-dimethyl-4-[2'-(1H-tetrazol-5-yl)biphenyl-4-yl]-methoxypyrimidine

Yield: 25%,
Melting point: 116-118 °C
$^1$H-NMR (DMSO-$d_6$)δ: 1.11 (3H, t, J = 7.6Hz), 1.26 (3H, t, J = 7.6Hz), 2.70 (2H, q, J = 7.5Hz), 2.76 (2H, q, J = 7.6Hz), 4.03 (2H, s), 5.43 (2H, s), 7.05 (2H, d, J = 8.3Hz), 7.24 (2H, d, J = 8.6Hz), 7.34-7.39 (2H, m), 7.50-7.78 (6H, m)
IR (KBr) $cm^{-1}$: 1605, 1570, 1420, 1345
MS (FAB) m/z: 521 (M + 1)

Reference Example 43: 5-(3-carboxybenzyl)-2,6-diethyl-3-[2'-(1H-tetrazol-5-yl)biphenyl-4-yl]methylpyrimidin-4-one

Yield: 21%,
Melting point: 125-127 °C
$^1$H-NMR (DMSO-$d_6$)δ: 1.08 (3H, t, J = 7.6Hz), 1.12 (3H, t, J = 7.3Hz), 2.54 (2H, q, J = 7.4Hz), 2.66 (2H, q, J = 7.2Hz), 3.94 (2H, s), 5.31 (2H, s), 7.09 (4H, s), 7.35-7.78 (8H, m)
IR (KBr) $cm^{-1}$: 2980, 1700, 1645, 1540
MS (FAB) m/z: 521 (M + 1)

Reference Example 44: 5-(3-carboxybenzyl)-6-ethyl-2-propyl-4-[2'-(1H-tetrazol-5-yl)biphenyl-4-yl]-methoxypyrimidine

Yield: 24%,
Melting point: 112-114 °C
$^1$H-NMR (DMSO-$d_6$)δ: 0.92 (3H, t, J = 7.4Hz), 1.11 (3H, t, J = 7.4Hz), 1.69-1.83 (2H, m), 2.69 (2H, q, J = 7.5Hz), 2.72 (2H, t, J = 7.4Hz), 4.03 (2H, s), 5.42 (2H, s), 7.05 (2H, d, J = 7.8Hz), 7.23 (2H, d, J = 8.1Hz), 7.34-7.41 (2H, m), 7.50-7.79 (6H, m)
IR (KBr) $cm^{-1}$: 3440, 1570, 1420, 1345
MS (FAB) m/z: 535 (M + 1)

Reference Example 45: 5-(3-carboxybenzyl)-6-ethyl-2-propyl-3-[2'-(1H-tetrazol-5-yl)biphenyl-4-yl]-methylpyrimidin-4-one

Yield: 16%,
Melting point: 123-125 °C
$^1$H-NMR (DMSO-$d_6$)δ: 0.87 (3H, t, J = 7.3Hz), 1.08 (3H, t, J = 7.6Hz), 1.59-1.68 (2H, m), 2.51-2.55 (2H, m), 2.61 (2H, t, J = 7.4Hz), 3.94 (2H, s), 5.31 (2H, s), 7.09 (4H, s), 7.38-7.79 (8H, m)
IR (KBr) $cm^{-1}$: 3440, 2970, 1650, 1540
MS (FAB) m/z: 535 (M + 1)

Reference Example 46: 2-butyl-5-(3-carboxybenzyl)-6-ethyl-4-[2'-(1H-tetrazol-5-yl)biphenyl-4-yl]-methoxypyrimidine

Yield: 22%,
Melting point: 99-100 °C
$^1$H-NMR (DMSO-$d_6$)δ: 0.89 (3H, t, J = 7.3Hz), 1.11 (3H, t, J = 7.4Hz), 1.27-1.41 (2H, m), 1.67-1.78 (2H,

m), 2.68 (2H, q,J =7.6Hz), 2.74 (2H, t, J=7.5Hz), 4.02 (2H, s), 5.41 (2H, s), 7.04 (2H, d, J=8.3Hz), 7.23 (2H, d, J=8.1Hz), 7.33-7.39 (2H, m), 7.49-7.77 (6H, m)

IR (KBr) $cm^{-1}$: 2960, 1570, 1420, 1345

MS (FAB) m/z: 549 (M+1)

Reference Example 47: 2-butyl-5-(3-carboxybenzyl)-6-ethyl-3-[2'-(1H-tetrazol-5-yl)biphenyl-4-yl]-methylpyrimidin-4-one

Yield: 25%,

Melting point: 108-110 °C

$^1$H-NMR (DMSO-$d_6$)δ: 0.82 (3H, t, J=7.3Hz), 1.07 (3H, t, J=7.6Hz), 1.24-1.33 (2H, m), 1.57-1.62 (2H, m), 2.52 (2H, q, J=7.3Hz), 2.61 (2H, t, J=7.4Hz), 3.94 (2H, s), 5.31 (2H, s), 7.09 (4H, s), 7.35-7.78 (8H, m)

IR (KBr) $cm^{-1}$: 2960, 1700, 1650, 1540

MS (FAB) m/z: 549 (M+1)

Reference Example 48: 5-(3-carboxybenzyl)-2-methyl-6-propyl-3-[2'-(1H-tetrazol-5-yl)biphenyl-4-yl]-methylpyrimidin-4-one

Yield: 6%,

Melting point: 144-146 °C

$^1$H-NMR (DMSO-$d_6$)δ: 0.89 (3H, t, J=7.3Hz), 1.54-1.63 (2H, m), 2.48 (3H, s), 2.81 (2H, t, J=7.3Hz), 4.07 (2H, s), 5.48 (2H, s), 7.03-7.22 (4H, m), 7.34-7.78 (8H, m)

IR (KBr) $cm^{-1}$: 2960, 1605, 1440, 1345

MS (FAB) m/z: 521 (M+1)

Reference Example 49: 5-(3-carboxybenzyl)-2-ethyl-6-propyl-4-[2'-(1H-tetrazol-5-yl)biphenyl-4-yl]-methoxypyrimidine

Yield: 29%,

Melting point: 130-132 °C

$^1$H-NMR (DMSO-$d_6$)δ: 0.85 (3H, t, J=7.4Hz), 1.25 (3H, t, J=7.5Hz), 1.55-1.62 (2H, m), 2.63-2.80 (4H, m), 4.03 (2H, s), 5.42 (2H, s), 7.05 (2H, d, J=7.8Hz), 7.24 (2H, d, J=8.0Hz), 7.34-7.39 (2H, m), 7.50-7.77 (6H, m)

IR (KBr) $cm^{-1}$: 2960, 1710, 1570, 1345

MS (FAB) m/z: 535 (M+1)

Reference Example 50: 5-(3-carboxybenzyl)-2-ethyl-6-propyl-3-[2'-(1H-tetrazol-5-yl)biphenyl-4-yl]-methylpyrimidin-4-one

Yield: 21%,

Melting point: 140-142 °C

$^1$H-NMR (DMSO-$d_6$)δ: 0.85 (3H, t, J=7.3Hz), 1.11 (3H, t, J=7.3Hz), 1.52-1.60 (2H, m), 2.48-2.53 (2H, m), 2.61-2.69 (2H, m), 3.94 (2H, s), 5.31 (2H, s), 7.09 (4H, s), 7.35-7.78 (8H, m)

IR (KBr) $cm^{-1}$: 3440, 2960, 1650, 1540

MS (FAB) m/z: 535 (M+1)

Reference Example 51: 5-(3-carboxybenzyl)-2,6-dipropyl-4-[2'-(1H-tetrazol-5-yl)biphenyl-4-yl]-methoxypyrimidine

Yield: 21%,

Melting point: 133-135 °C

$^1$H-NMR (DMSO-$d_6$)δ: 0.86 (3H, t, J=7.3Hz), 0.92 (3H, t, J=7.4Hz), 1.51-1.65 (2H, m), 1.69-1.83 (2H, m), 2.51-2.78 (4H, m), 4.03 (2H, s), 5.42 (2H, s), 7.05 (2H, d, J=7.6Hz), 7.22 (2H, d, J=8.2Hz), 7.33-7.36 (2H, m), 7.50-7.78 (6H, m)

IR (KBr) $cm^{-1}$: 2960, 1710, 1570, 1420

MS (FAB) m/z: 549 (M+1)

Reference Example 52: 5-(3-carboxybenzyl)-2,6-dipropyl-3-[2'-(1H-tetrazol-5-yl)biphenyl-4-yl]-methylpyrimidin-4-one

Yield: 26%,
Melting point: 128-130 ° C
$^1$H-NMR (DMSO-$d_6$)δ: 0.83 (3H, t, J = 7.3Hz), 0.86 (3H, t, J = 7.3Hz), 1.51-1.66 (4H, m), 2.51 (2H, t, J = 7.3Hz), 2.61 (2H, t, J = 7.2Hz), 3.94 (2H, s), 5.31 (2H, s), 7.09 (4H, s), 7.35-7.58 (4H, m), 7.63-7.68 (2H, m), 7.73-7.78 (2H, m)
IR (KBr) $cm^{-1}$: 2960, 2930, 1655, 1540
MS (FAB) m/z: 549 (M + 1)

Reference Example 53: 2-butyl-5-(3-carboxybenzyl)-6-propyl-4-[2'-(1H-tetrazol-5-yl)biphenyl-4-yl]-methoxypyrimidine

Yield: 24%,
Melting point: 90-92 ° C
$^1$H-NMR (DMSO-$d_6$)δ: 0.86 (3H, t, J = 7.6Hz), 0.89 (3H, t, J = 7.3Hz), 1.30-1.38 (2H, m), 1.54-1.62 (2H, m), 1.70-1.76 (2H, m), 2.71 (2H, t, J = 7.6Hz), 2.79 (2H, t, J = 7.6Hz), 4.04 (2H, s), 5.44 (2H, s), 7.03-7.07 (2H, m), 7.20-7.26 (2H, m), 7.34-7.36 (2H, m), 7.49-7.77 (6H, m)
IR (KBr) $cm^{-1}$: 3440, 2960, 1570, 1345
MS (FAB) m/z: 563 (M + 1)

Reference Example 54: 2-butyl-5-(3-carboxybenzyl)-6-propyl-3-[2'-(1H-tetrazol-5-yl)biphenyl-4-yl]-methylpyrimidin-4-one

Yield: 22%,
Melting point: 103-105 ° C
$^1$H-NMR (DMSO-$d_6$)δ: 0.81 (3H, t, J = 7.3Hz), 0.84 (3H, t, J = 7.3Hz), 1.23-1.31 (2H, m), 1.51-1.61 (4H, m), 2.50 (2H, t, J = 7.4Hz), 2.62 (2H, t,J = 7.4Hz), 3.94 (2H, s), 5.31 (2H, s), 7.09 (4H, s), 7.35-7.78 (8H, m)
IR (KBr) $cm^{-1}$: 2960, 1705, 1650, 1540
MS (FAB) m/z: 563 (M + 1)

Reference Example 55: 5-(4-carboxybenzyl)-6-ethyl-2-methyl-4-[2'-(1H-tetrazol-5-yl)biphenyl-4-yl]-methoxypyrimidine

Yield: 10%,
Melting point: 122-123 ° C
$^1$H-NMR (DMSO-$d_6$)δ: 1.10 (3H, t, J = 7.4Hz), 2.49 (3H, s), 2.66 (2H, q, J = 7.5Hz), 4.02 (2H, s), 5.40 (2H, s), 7.04-7.17 (2H, m), 7.22 (2H, d, J = 8.1Hz), 7.25-7.36 (2H, m), 7.50-7.68 (4H, m), 7.81 (2H, d, J = 8.1Hz)
IR (KBr) $cm^{-1}$: 1610, 1575, 1420, 1345
MS (FAB) m/z: 507 (M + 1)

Reference Example 56: 5-(4-carboxybenzyl)-6-ethyl-2-methyl-3-[2'-(1H-tetrazol-5-yl)biphenyl-4-yl]-methylpyrimidin-4-one

Yield: 48%,
Melting point: 175-176 ° C
$^1$H-NMR (DMSO-$d_6$)δ: 1.05 (3H, t, J = 7.4Hz), 2.39 (3H, s), 2.50 (2H, q, J = 7.4Hz), 3.94 (2H, s), 5.28 (2H, s), 7.10 (4H, s), 7.29 (2H, d, J = 8.3Hz), 7.49-7.57 (2H, m), 7.62-7.67 (2H, m), 7.83 (2H, d, J = 8.3Hz)
IR (KBr) $cm^{-1}$: 1705, 1650, 1610, 1540
MS (FAB) m/z: 507 (M + 1)

Reference Example 57: Preparation of 5-(3-carboxybenzyl)-6-ethyl-2-methyl-4-[2'-(1H-tetrazol-5-yl)biphenyl-4-yl]methoxypyrimidine

Yield: 14%,
Melting point: 161-163 ° C (decomposition)
$^1$H-NMR (DMSO-$d_6$)δ: 1.05 (3H, t, J = 7.6Hz), 2.48 (3H, s), 2.68 (2H, q, J = 7.6Hz), 4.02 (2H, s), 5.40 (2H,

s), 7.04 (2H, d, J = 8.3Hz), 7.22 (2H, d, J = 8.3Hz), 7.33-7.38 (2H, m), 7.51-7.56 (6H, m)
IR (KBr) $cm^{-1}$: 1605, 1570, 1425, 1345
MS (FAB) m/z: 507 (M + 1)

Reference Example 58: Preparation of 5-(3-carboxybenzyl)-6-ethyl-2-methyl-4-[2'-(triphenylmethyltetrazol-5-yl)biphenyl-4-yl]methoxypyrimidine

(a) Preparation of 4-chloro-6-ethyl-5-(3-methoxycarbonylbenzyl)-2-methylpyrimidine

In phosphorus oxychloride (70 ml), 6-ethyl-5-(3-methoxycarbonylbenzyl)-2-methylpyrimidin-4(3H)-one (13.3 g) prepared in Reference Example 12 was dissolved. To the solution, triethylamine (6.4 ml) was added. Then, the whole was stirred at 45 °C for 30 minutes and concentrated under reduced pressure. Toluene was added to the residue and the mixture was concentrated. The procedure was repeated three times. To the residue, toluene was added, and the mixture was washed with water, saturated saline, and 5% sodium hydrogencarbonate solution successively. The mixture was dried over anhydrous magnesium sulfate. The solvent was evaporated under reduced pressure to obtain 4-chloro-6-ethyl-5-(3-methoxycarbonylbenzyl)-2-methylpyrimidine (12.9 g) as brown oil.

Yield: 92%

$^1$H-NMR ($CDCl_3$)δ: 1.37 (3H, t, J = 7.6Hz), 2.48 (3H, s), 2.93 (2H, q, J = 7.6Hz), 3.90 (3H, s), 4.21 (2H, s), 7.29 (2H, d, J = 7.6Hz), 7.37 (2H, t, J = 7.6Hz), 7.83 (1H, s), 7.91 (1H, d, J = 7.6Hz)
IR (KBr) $cm^{-1}$: 1725, 1565, 1525, 1435, 1285
MS (FAB) m/z: 305 (M + 1)

(b) Preparation of 5-(3-carboxybenzyl)-6-ethyl-2-methyl-4-[2'-(triphenylmethyltetrazol-5-yl)biphenyl-4-yl]-methoxypyrimidine

To a suspension of sodium hydride (1.68 g) in dry N,N-dimethylformamide (40 ml), 4'-hydroxymethyl-2-(triphenylmethyltetrazol-5-yl)biphenyl (19.8 g) was added portionwise under stirring at room temperature. The mixture was stirred for 30 minutes. In dry N,N-dimethylformamide (20 ml), the 4-chloro-6-ethyl-5-(3-methoxycarbonylbenzyl)-2-methylpyrimidine (12.9 g) prepared in Reference Example 58 (a) was dissolved and the resulting solution was added dropwise to the reaction mixture. The whole was stirred at room temperature overnight. After the reaction was completed, the reaction mixture was concentrated under reduced pressure and water (100 ml) was added. Then, extraction was performed with ethyl acetate (100 ml) twice. The ethyl acetate extract layer was dried over anhydrous magnesium sulfate and concentrated under reduced pressure. Then, methanol (80 ml) and concentrated hydrochloric acid (4 ml) were added to the residue and the mixture was stirred at room temperature for 1 hour. To the reaction mixture, 5N NaOH solution (40 ml) was added. The whole was stirred at 80 °C for 2 hours and concentrated under reduced pressure. Water (100 ml) was added and the whole was washed with diethyl ether (100 ml) twice. After the pH of the resulting solution was adjusted to 4 with 1N hydrochloric acid, the solution was extracted with ethyl acetate (100 ml) twice. The ethyl acetate extract layer was dried over anhydrous magnesium sulfate and concentrated under reduced pressure. The resulting residue was dissolved in dichloromethane (100 ml), and triphenylmethylchloride (11.2 g) and triethylamine (5.5 ml) were added to the solution. The whole was stirred at room temperature overnight. After the reaction was completed, the reaction mixture was washed with water and dried over anhydrous magnesium sulfate. The solvent was evaporated under reduced pressure and the resulting residue was purified, by silica gel column chromatography, eluting with chloroform/methanol (50:1) to obtain the above-titled compound (11.4 g).

Yield: 38%,

Melting point: 101-103 °C

$^1$H-NMR ($CDCl_3$)δ: 1.17 (3H, t, J = 7.6Hz), 2.62 (3H, s), 2.76 (2H, q, J = 7.6Hz), 3.99 (2H, s), 5.33 (2H, s), 6.86-6.92 (6H, m), 7.01-7.50 (18H, m), 7.89-7.97 (3H, m)
IR (KBr) $cm^{-1}$: 3700-3200, 1720, 1575, 1445
MS (FAB) m/z: 749 (M + 1)

Example 1: Preparation of 5-(4-hydroxymethylbenzyl)-6-methyl-2-propyl-4-[2'-(1H-tetrazol-5-yl)biphenyl-4-yl]methoxypyrimidine

(a) Preparation of 5-(4-hydroxymethylbenzyl)-6-methyl-2-propyl-4-[2'-(triphenylmethyltetrazol-5-yl)biphenyl-4-yl]methoxypyrimidine

To a solution of the compound (0.84 g) prepared in Reference Example 21 in dichloromethane (20 ml), triphenylmethyl chloride (0.50 g) and triethylamine (0.26 ml) were added. The mixture was stirred at room temperature overnight. After the reaction was completed, the reaction mixture was washed with water and dried over anhydrous sodium sulfate. The solvent was evaporated and the resulting residue was purified, by silica gel column chromatography, eluting with dichloromethane/methanol (30:1) to obtain N-trityl compound (0.90 g) as amorphous (yield = 73%). The resulting N-trityl compound was dissolved in dry tetrahydrofuran (THF) (8 ml) and the solution was stirred at -10 °C for 10 minutes. After N-methylmorpholine (0.14 ml) and ethyl chloroformate (0.12 ml) were added to the solution at the same temperature, the whole was further stirred for 10 minutes. After stirring, sodium borohydride (0.14 g) was added quickly at the same temperature, and the reaction mixture was warmed to 0 °C. Then, dry methanol (16 ml) was added portionwise to the reaction mixture at the same temperature. After stirring for 15 minutes, the reacton mixture was concentrated and water (20 ml) was added. The whole was extracted with ethyl acetate (150 ml) twice. The ethyl acetate extract layer was washed with water and saturated saline and dried over anhydrous sodium sulfate. The solvent was evaporated under reduced pressure and the resulting residue was purified, by silica gel column chromatography, eluting with n-hexane/ethyl acetate (2:1) to obtain the above-titled compound (0.78 g) as amorphous.

Yield: 88%,

Melting point: 68-70 °C

$^1$H-NMR ($CDCl_3$)δ: 1.00 (3H, t, J = 7.3Hz), 1.79-1.88 (2H, m), 2.41 (3H, s), 2.78 (2H, t, J = 7.7Hz), 3.93 (2H, s), 4.61 (2H, s), 5.36 (2H, s), 6.87-6.91 (6H, m), 7.06-7.09 (6H, m), 7.18-7.52 (14H, m), 7.88-7.91 (1H, m)

IR (KBr) $cm^{-1}$: 3405, 2960, 2925, 1570

MS (FAB) m/z: 749 (M + 1)

(b) Preparation of 5-(4-hydroxymethylbenzyl)-6-methyl-2-propyl-4-[2'-(1H-tetrazol-5-yl)biphenyl-4-yl]-methoxypyrimidine

The compound (0.36 g) prepared in Example 1 (a) was dissolved in methanol (10 ml) and a few drops of concentrated hydrochloric acid were added to the solution. The mixture was stirred at room temperature for 4 hours. After 1N NaOH (2 ml) was added, the reaction mixture was concentrated to a solid under reduced pressure. Water (20 ml) was added to dissolve the resulting residue. The insolubles were filtered out. The pH of the resulting solution was adjusted to 4 with 1N hydrochloric acid. The precipitated crystals were collected by filtration to obtain the above-titled compound (0.22 g) as white powder.

Yield: 90%,

Melting point: 83-85 °C

$^1$H-NMR (DMSO-$d_6$)δ: 0.91 (3H, t, J = 7.3Hz), 1.75 (2H, q, J = 7.4Hz), 2.37 (3H, s), 2.70 (2H, t, J = 7.4Hz), 3.93 (2H, s), 4.44 (2H, s), 5.43 (2H, s), 7.04-7.09 (4H, m), 7.17-7.39 (4H, m), 7.50-7.70 (4H, m)

IR (KBr) $cm^{-1}$: 2960, 1570, 1420, 1345

MS (FAB) m/z: 507 (M + 1)

Example 2: Preparation of 5-(4-methoxymethylbenzyl)-6-methyl-2-propyl-4-[2'-(1H-tetrazol-5-yl)biphenyl-4-yl]methoxypyrimidine

(a) Preparation of 5-(4-methoxymethylbenzyl)-6-methyl-2-propyl-4-[2'-(triphenylmethyltetrazol-5-yl)biphenyl-4-yl]methoxypyrimidine

The compound (0.39 g) prepared in Example 1 (a) was dissolved in dry N,N-dimethylformamide (10 ml) under an argon gas atmosphere. The mixture was stirred at room temperature for 10 minutes. Then, sodium hydride (0.02 g) was added to the reaction mixture. After stirring for 10 minutes, methyl iodide (0.10 ml) was added to the mixture and the whole was stirred at room temperature for 30 hours. After the reaction was completed, water (40 ml) was added to the reaction mixture. The whole was extracted with ethyl acetate (30 ml) twice. The ethyl acetate extract layer was washed with water and saturated saline and dried

over anhydrous sodium sulfate. The solvent was evaporated under reduced pressure and the resulting residue was purified, by silica gel column chromatography, eluting with n-hexane/ethyl acetate (3:1) to obtain the above-titled compound (0.23 g) as amorphous.

Yield: 58%,

Melting point: 52-54 °C

$^1$H-NMR ($CDCl_3$)δ: 1.00 (3H, t, J=7.4Hz), 1.77-1.93 (2H, m), 2.40 (3H, s), 2.78 (2H, t, J=7.7Hz), 3.34 (3H, s), 3.93 (2H, s), 4.38 (2H, s), 5.34 (2H, s), 6.87-6.97 (6H, m), 7.07-7.10 (6H, m), 7.18-7.53 (14H, m), 7.91-7.95 (1H, m)

IR (KBr) $cm^{-1}$: 1575, 1445, 1415, 1340

MS (FAB) m/z: 763 (M+1)

(b) Preparation of 5-(4-methoxymethylbenzyl)-6-methyl-2-propyl-4-[2'-(1H-tetrazol-5-yl)biphenyl-4-yl]-methoxypyrimidine

The compound (0.22 g) prepared in Example 2 (a) was treated in accordance with the method described in Example 1 (b) to obtain the above-titled compound (0.14 g).

Yield: 96%,

Melting point: 130-131 °C

$^1$H-NMR (DMSO-$d_6$)δ: 0.91 (3H, t, J=7.3Hz), 1.70-1.78 (2H, m), 2.35 (3H, s), 2.68 (2H, t, J=7.4Hz), 3.25 (3H, s), 3.93 (2H, s), 4.34 (2H, s), 5.41 (2H, s), 7.04-7.09 (4H, m), 7.17 (2H, d, J=8.1Hz), 7.24 (2H, d, J=8.3Hz), 7.50-7.69 (4H, m)

IR (KBr) $cm^{-1}$: 1570, 1415, 1345, 1100

MS (FAB) m/z: 521 (M+1)

Examples 3 and 4

The compound prepared in Reference Example 22 was treated in accordance with the methods described in above-mentioned Examples 1 and 2 to obtain the compounds described in the following Examples 3 and 4.

Example 3: Preparation of 5-(4-hydroxymethylbenzyl)-6-methyl-2-propyl-3-[2'-(1H-tetrazol-5-yl)biphenyl-4-yl]methylpyrimidin-4-one

Yield: 64%,

Melting point: 108-110 °C

$^1$H-NMR (DMSO-$d_6$)δ: 0.85 (3H, t, J=7.3Hz), 1.55-1.63 (2H, m), 2.23 (3H, s), 2.59 (2H, t, J=7.6Hz), 3.83 (2H, s), 4.44 (2H, s), 5.30 (2H, s), 7.08 (4H, s), 7.13-7.35 (4H, m), 7.50-7.66 (4H, m)

IR (KBr) $cm^{-1}$: 3420, 2960, 1645, 1540

MS (FAB) m/z: 507 (M+1)

Example 4: Preparation of 5-(4-methoxymethylbenzyl)-6-methyl-2-propyl-3-[2'-(1H-tetrazol-5-yl)biphenyl-4-yl]methylpyrimidin-4-one

Yield: 43%,

Melting point: 87-89 °C

$^1$H-NMR (DMSO-$d_6$)δ: 0.85 (3H, t, J=7.3Hz), 1.56-1.64 (2H, m), 2.22 (3H, s), 2.58 (2H, t, J=7.4Hz), 3.26 (3H, s), 3.84 (2H, s), 4.35 (2H, s), 5.29 (2H, s), 7.07 (4H, s), 7.16-7.39 (4H, m), 7.50-7.69 (4H, m)

IR (KBr) $cm^{-1}$: 2960, 1650, 1540, 1450

MS (FAB) m/z: 521 (M+1)

Example 5: Preparation of 5-(4-hydroxymethylbenzyl)-2,6-dimethyl-3-[2'-(1H-tetrazol-5-yl)biphenyl-4-yl]-methylpyrimidin-4-one

The compound prepared in Reference Example 23 was treated in accordance with the method described in above-mentioned Example 1 to obtain the above-titled compound.

Yield: 8%,

Melting point: 129 °C (decomposition)

$^1$H-NMR (DMSO-$d_6$)δ: 2.21 (3H, s), 2.38 (3H, s), 3.92 (2H, s), 4.44 (2H, s), 5.28 (2H, s), 7.09 (4H, s),

7.13-7.33 (3H, m), 7.50-7.69 (4H, m), 7.83-7.86 (1H, m)
IR (KBr) $cm^{-1}$: 1705, 1650, 1610, 1540
MS (FAB) m/z: 479 (M + 1)

Example 6: Preparation of 2,6-diethyl-5-(4-hydroxymethylbenzyl)-4-[2'-(1H-tetrazol-5-yl)biphenyl-4-yl]-methoxypyrimidine

The compound prepared in Reference Example 24 was treated in accordance with the method described in above-mentioned Example 1 to obtain the above-titled compound.
Yield: 51%,
Melting point: 102-104 °C
$^1$H-NMR (DMSO-$d_6$)δ: 1.10 (3H, t, J = 7.6Hz), 1.25 (3H, t, J = 7.6Hz), 2.66 (2H, q, J = 7.5Hz), 2.75 (2H, q, J = 7.4Hz), 3.94 (2H, s), 4.44 (2H, s), 5.42 (2H, s), 7.02-7.14 (4H, m), 7.18 (2H, d, J = 7.8Hz), 7.25 (2H, d, J = 8.3Hz), 7.51-7.69 (4H, m)
IR (KBr) $cm^{-1}$: 3400, 1570, 1420, 1345
MS (FAB) m/z: 507 (M + 1)

Example 7: Preparation of 2,6-diethyl-5-(4-hydroxymethylbenzyl)-3-[2'-(1H-tetrazol-5-yl)biphenyl-4-yl]-methylpyrimidin-4-one

The compound prepared in Reference Example 25 was treated in accordance with the method described in above-mentioned Example 1 to obtain the above-titled compound.
Yield: 51%,
Melting point: 109-111 °C
$^1$H-NMR(DMSO-$d_6$)δ: 1.09 (3H, t, J = 7.4Hz), 1.12 (3H, t, J = 7.3Hz), 2.53 (2H, q, J = 7.6Hz), 2.66 (2H, q, J = 7.2Hz), 3.85 (2H, s), 4.44 (2H, s), 5.29 (2H, s), 7.08 (4H, s), 7.12-7.21 (4H, m), 7.50-7.69 (4H, m)
IR (KBr) $cm^{-1}$: 3440, 2980, 1645, 1540
MS (FAB) m/z: 507 (M + 1)

Example 8: Preparation of 6-ethyl-5-(4-hydroxymethylbenzyl)-2-propyl-4-[2'-(1H-tetrazol-5-yl)biphenyl-4-yl]-methoxypyrimidine

The compound prepared in Reference Example 26 was treated in accordance with the method described in above-mentioned Example 1 to obtain the above-titled compound.
Yield: 60%,
Melting point: 71-73 °C
$^1$H-NMR (DMSO-$d_6$)δ: 0.92 (3H, t, J = 7.3Hz), 1.10 (3H, t, J = 7.5Hz), 1.69-1.82 (2H, m), 2.62-2.73 (4H, m), 3.94 (2H, s), 4.44 (2H, s), 5.41 (2H, s), 7.02-7.11 (4H, m), 7.19 (2H, d, J = 8.2Hz), 7.25 (2H, d, J = 8.3Hz), 7.50-7.69 (4H, m)
IR (KBr) $cm^{-1}$: 3400, 1570, 1420, 1345
MS (FAB) m/z: 521 (M + 1)

Example 9: Preparation of 6-ethyl-5-(4-hydroxymethylbenzyl)-2-propyl-3-[2'-(1H-tetrazol-5-yl)biphenyl-4-yl]-methylpyrimidin-4-one

The compound prepared in Reference Example 27 was treated in accordance with the method described in above-mentioned Example 1 to obtain the above-titled compound.
Yield: 42%,
Melting point: 102-104 °C
$^1$H-NMR (DMSO-$d_6$)δ: 0.86 (3H, t, J = 7.3Hz), 1.07 (3H, t, J = 7.4Hz), 1.59-1.67 (2H, m), 2.50-2.54 (2H, m), 2.60 (2H, t, J = 7.3Hz), 3.85 (2H, s), 4.44 (2H, s), 5.29 (2H, s), 7.08 (4H, s), 7.13 (2H, d, J = 8.1Hz), 7.20 (2H, d, J = 8.3Hz), 7.51-7.69 (4H, m)
IR (KBr) $cm^{-1}$: 3440, 2960, 1650, 1540
MS (FAB) m/z: 521 (M + 1)

Example 10: Preparation of 2-butyl-6-ethyl-5-(4-hydroxymethylbenzyl)-4-[2'-(1H-tetrazol-5-yl)biphenyl-4-yl]-methoxypyrimidine

The compound prepared in Reference Example 28 was treated in accordance with the method described in above-mentioned Example 1 to obtain the above-titled compound.

Yield: 45%,

Melting point: 73-75 °C

$^1$H-NMR (DMSO-$d_6$)δ: 0.89 (3H, t, J = 7.3Hz), 1.10 (3H, t, J = 7.6Hz), 1.27-1.41 (2H, m), 1.67-1.78 (2H, m), 2.63-2.69 (2H, m), 2.74 (2H, t, J = 7.4Hz), 3.94 (2H, s), 4.44 (2H, s), 5.42 (2H, s), 7.02-7.08 (4H, m), 7.18 (2H, d, J = 8.1Hz), 7.25 (2H, d, J = 8.1Hz), 7.51-7.69 (4H, m)

IR (KBr) $cm^{-1}$: 2960, 1570, 1420, 1345

MS (FAB) m/z: 535 (M + 1)

Example 11: Preparation of 5-(4-hydroxymethylbenzyl)-6-methyl-2-pentyl-4-[2'-(1H-tetrazol-5-yl)biphenyl-4-yl]methoxypyrimidine

The compound prepared in Reference Example 29 was treated in accordance with the method described in above-mentioned Example 1 to obtain the above-titled compound.

Yield: 41%,

Melting point: 95-97 °C

$^1$H-NMR (DMSO-$d_6$)δ: 0.87 (3H, t, J = 7.1Hz), 1.30-1.38 (4H, m), 1.71-1.82 (2H, m), 2.50 (3H, s), 2.87 (2H, t, J = 7.6Hz), 3.96 (2H, s), 4.45 (2H, s), 5.52 (2H, s), 7.07-7.42 (8H, m), 7.47-7.70 (4H, m)

IR (KBr) $cm^{-1}$: 3600-3200, 2960, 1605, 1470, 1345

MS (FAB) m/z: 535 (M + 1)

Example 12: Preparation of 5-(4-hydroxymethylbenzyl)-6-methyl-2-pentyl-3-[2'-(1H-tetrazol-5-yl)biphenyl-4-yl]methylpyrimidin-4-one

The compound prepared in Reference Example 30 was treated in accordance with the method described in above-mentioned Example 1 to obtain the above-titled compound.

Yield: 6%,

Melting point: 113-115 °C

$^1$H-NMR (DMSOv)δ: 0.81 (3H, t, J = 7.1Hz), 1.21-1.26 (4H, m), 1.54-1.59 (2H, m), 2.25 (3H, s), 2.65 (2H, t, J = 7.6Hz), 3.83 (2H, s), 4.44 (2H, s), 5.30 (2H, s), 7.08-7.44 (8H, m), 7.48-7.66 (4H, m)

IR (KBr) $cm^{-1}$: 3600-3300, 2960, 1700, 1655, 1540

MS (FAB) m/z: 535 (M + 1)

Example 13: Preparation of 5-(3-hydroxymethylbenzyl)-2,6-dimethyl-4-[2'-(1H-tetrazol-5-yl)biphenyl-4-yl]-methoxypyrimidine

The compound prepared in Reference Example 31 was treated in accordance with the method described in above-mentioned Example 1 to obtain the above-titled compound.

Yield: 43%,

Melting point: 78 °C (decomposition)

$^1$H-NMR (DMSO-$d_6$)δ: 2.45 (3H,s), 2.56 (3H, s), 3.96 (2H, s), 4.41 (2H, s), 5.48 (2H, s), 6.96-7.28 (8H, m), 7.51-7.66 (4H, m)

IR (KBr) $cm^{-1}$: 3600-3200, 2930, 1635, 1345

MS (FAB) m/z: 479 (M + 1)

Example 14: Preparation of 5-(3-hydroxymethylbenzyl)-2,6-dimethyl-3-[2'-(1H-tetrazol-5-yl)biphenyl-4-yl]-methylpyrimidin-4-one

The compound prepared in Reference Example 32 was treated in accordance with the method described in above-mentioned Example 1 to obtain the above-titled compound.

Yield: 35%,

Melting point: 105 °C (decomposition)

$^1$H-NMR (DMSO-$d_6$)δ: 2.20 (3H, s), 2.36 (3H, s), 3.84 (2H, s), 4.44 (2H, s), 5.28 (2H, s), 7.04-7.22 (8H, m), 7.50-7.68 (4H, m)

IR (KBr) $cm^{-1}$: 3600-3300, 2930, 1700, 1655, 1545
MS (FAB) m/z: 479 (M + 1)

Example 15: Preparation of 2-ethyl-5-(3-hydroxymethylbenzyl)-6-methyl-4-[2'-(1H-tetrazol-5-yl)biphenyl-4-yl]methoxypyrimidine

The compound prepared in Reference Example 33 was treated in accordance with the method described in above-mentioned Example 1 to obtain the above-titled compound.

Yield: 23%,
Melting point: 89 ° C (decomposition)
$^1$H-NMR (DMSO-$d_6$)δ: 1.24 (3H, t, J = 7.5Hz), 2.37 (3H, s), 2.75 (2H, q, J = 7.5Hz), 3.94 (2H, s), 4.42 (2H, s), 5.44 (2H, s), 6.95-7.36 (8H, m), 7.50-7.74 (4H,m)
IR (KBr) $cm^{-1}$: 3600-3300, 2940, 1610, 1575, 1345
MS (FAB) m/z: 493 (M + 1)

Example 16: Preparation of 2-ethyl-5-(3-hydroxymethylbenzyl)-6-methyl-3-[2'-(1H-tetrazol-5-yl)biphenyl-4-yl]methylpyrimidin-4-one

The compound prepared in Reference Example 34 was treated in accordance with the method described in above-mentioned Example 1 to obtain the above-titled compound.

Yield: 20%,
Melting point: 95 ° C (decomposition)
$^1$H-NMR (DMSO-$d_6$)δ: 1.09 (3H, t, J = 7.3Hz), 2.22 (3H, s), 2.63 (2H, q, J = 7.3Hz), 3.85 (2H, s), 4.44 (2H, s), 5.30 (2H, s), 7.07-7.22 (8H, m), 7.50-7.68 (4H, m)
IR (KBr) $cm^{-1}$: 3600-3300, 2940, 1650, 1540
MS (FAB) m/z: 493 (M + 1)

Examples 17 and 18

The compound prepared in Reference Example 35 was treated in accordance with the method described in above-mentioned Examples 1 and 2 to obtain the compounds described in the following Examples 17 and 18.

Example 17: Preparation of 5-(3-hydroxymethylbenzyl)-6-methyl-2-propyl-4-[2'-(1H-tetrazol-5-yl)biphenyl-4-yl]methoxypyrimidine

Yield: 32%,
Melting point: 177-178 ° C
$^1$H-NMR (DMSO-$d_6$)δ: 0.91 (3H, t, J = 7.3Hz), 1.67-1.78 (2H, m), 2.35 (3H, s), 2.68 (2H, t, J = 7.4Hz), 3.93 (2H, s), 4.41 (2H, s), 5.42 (2H, s), 7.07 (2H, d, J = 8.3Hz), 7.10-7.21 (4H, m), 7.27 (2H, d, J = 8.5Hz), 7.50-7.69 (4H, m)
IR (KBr) $cm^{-1}$: 3400, 1570, 1420, 1345
MS (FAB) m/z: 507 (M + 1)

Example 18: Preparation of 5-(3-methoxymethylbenzyl)-6-methyl-2-propyl-4-[2'-(1H-tetrazol-5-yl)biphenyl-4-yl]methoxypyrimidine

Yield: 67%,
Melting point: 151-153 ° C
$^1$H-NMR (DMSO-$d_6$)δ: 0.91 (3H, t, J = 7.3Hz), 1.70-1.78 (2H, m), 2.35 (3H, s), 2.68 (2H, t, J = 7.4Hz), 3.23 (3H, s), 3.94 (2H, s), 4.31 (2H, s), 5.42 (2H, s), 6.99-7.20 (4H, m), 7.06 (2H, d, J = 8.3Hz), 7.26 (2H, d, J = 8.3Hz), 7.50-7.66 (4H, m)
IR (KBr) $cm^{-1}$: 1575, 1420, 1345, 1100
MS (FAB) m/z: 521 (M + 1)

Examples 19 and 20

The compound prepared in Reference Example 36 was treated in accordance with the method described in above-mentioned Examples 1 and 2 to obtain the compounds described in the following Examples 19 and 20.

Example 19: Preparation of 5-(3-hydroxymethylbenzyl)-6-methyl-2-propyl-3-[2'-(1H-tetrazol-5-yl)biphenyl-4-yl]methylpyrimidin-4-one

Yield: 65%,
Melting point: 97-99 ° C
$^1$H-NMR (DMSO-$d_6$)δ: 0.85 (3H, t, J = 7.5Hz), 1.56-1.64 (2H, m), 2.22 (3H, s), 2.58 (2H, t, J = 7.5Hz), 3.84 (2H, s), 4.44 (2H, s), 5.30 (2H, s), 7.08 (4H, s), 7.12-7.23 (4H, m), 7.50-7.69 (4H, m)
IR (KBr) $cm^{-1}$: 2960, 1650, 1535, 1445
MS (FAB) m/z: 507 (M + 1)

Example 20: Preparation of 5-(3-methoxymethylbenzyl)-6-methyl-2-propyl-3-[2'-(1H-tetrazol-5-yl)biphenyl-4-yl]methylpyrimidin-4-one

Yield: 48%,
Melting point: 83-85 ° C
$^1$H-NMR (DMSO-$d_6$)δ: 0.85 (3H, t, J = 7.3Hz), 1.56-1.64 (2H, m), 2.22 (3H, s), 2.58 (2H, t, J = 7.4Hz), 3.26 (3H, s), 3.85 (2H, s), 4.35 (2H, s), 5.30 (2H, s), 7.07 (4H, s), 7.12-7.26 (4H, m), 7.50-7.69 (4H, m)
IR (KBr) $cm^{-1}$: 3410, 2920, 1650, 1540
MS (FAB) m/z: 521 (M + 1)

Examples 21 and 22

The compound prepared in Reference Example 37 was treated in accordance with the method described in above-mentioned Examples 1 and 2 to obtain the compounds described in the following Examples 21 and 22.

Example 21: Preparation of 2-butyl-5-(3-hydroxymethylbenzyl)-6-methvl-4-[2'-(1H-tetrazol-5-yl)biphenyl-4-yl]methoxypyrimidine

Yield: 74%,
Melting point: 81-83 ° C
$^1$H-NMR (DMSO-$d_6$)δ: 0.89 (3H, t, J = 7.3Hz), 1.26-1.37 (2H, m), 1.65-1.76 (2H, m), 2.35 (3H, s), 2.71 (2H, t, J = 7.4Hz), 3.93 (2H, s), 4.41 (2H, s), 5.42 (2H, s), 6.95-7.29 (8H, m), 7.50-7.69 (4H, m)
IR (KBr) $cm^{-1}$: 2955, 1570, 1420, 1345
MS (FAB) m/z: 521 (M + 1)

Example 22: Preparation of 2-butyl-5-(3-methoxymethylbenzyl)-6-methyl-4-[2'-(1H-tetrazol-5-yl)biphenyl-4-yl]methoxypyrimidine

Yield: 26%,
Melting point: 74-76 ° C
$^1$H-NMR (DMSO-$d_6$)δ: 0.88 (3H, t, J = 7.2Hz), 1.26-1.37 (2H, m), 1.65-1.73 (2H, m), 2.35 (3H, s), 2.71 (2H, t, J = 7.6Hz), 3.23 (3H, s), 3.94 (2H, s), 4.31 (2H, s), 5.42 (2H, s), 6.99-7.27 (8H, m), 7.50-7.69 (4H, m)
IR (KBr) $cm^{-1}$: 1575, 1420, 1345, 1090
MS (FAB) m/z: 535 (M + 1)

Examples 23 and 24

The compound prepared in Reference Example 38 was treated in accordance with the method described in above-mentioned Examples 1 and 2 to obtain the compounds described in the following Examples 23 and 24.

Example 23: Preparation of 2-butyl-5-(3-hydroxymethylbenzyl)-6-methyl-3-[2'-(1H-tetrazol-5-yl)biphenyl-4-yl]methylpyrimidin-4-one

Yield: 66%,
Melting point: 91-93 ° C
$^1$H-NMR (DMSO-$d_6$)δ: 0.81 (3H, t, J = 7.2Hz), 1.23-1.31 (2H, m), 1.53-1.58 (2H, m), 2.22 (3H, s), 2.60 (2H, t, J = 7.6Hz), 3.84 (2H, s), 4.44 (2H, s), 5.30 (2H, s), 7.08 (4H, s), 7.05-7.23 (4H, m), 7.50-7.66 (4H, m)
IR (KBr) $cm^{-1}$: 3400, 2960, 1650, 1535
MS (FAB) m/z: 521 (M + 1)

Example 24: Preparation of 2-butyl-5-(3-methoxymethylbenzyl)-6-methyl-3-[2'-(1H-tetrazol-5-yl)biphenyl-4-yl]methylpyrimidin-4-one

Yield: 14%,
Melting point: 80-82 ° C
$^1$H-NMR (DMSO-$d_6$)δ: 0.81 (3H, t, J = 7.2Hz), 1.23-1.31 (2H, m), 1.50-1.58 (2H, m), 2.21 (3H, s), 2.60 (2H, t, J = 7.6Hz), 3.26 (3H, s), 3.85 (2H, s), 4.35 (2H, s), 5.30 (2H, s), 7.07 (4H, s), 7.08-7.26 (4H, m), 7.49-7.69 (4H, m)
IR (KBr) $cm^{-1}$: 3440, 2925, 1650, 1540
MS (FAB) m/z: 535 (M + 1)

Example 25: Preparation of 5-(3-hydroxymethylbenzyl)-6-methyl-2-pentyl-4-[2'-(1H-tetrazol-5-yl)biphenyl-4-yl]methoxypyrimidine

The compound prepared in Reference Example 39 was treated in accordance with the method described in above-mentioned Example 1 to obtain the above-titled compound.
Yield: 16%,
Melting point: 74-76 ° C
$^1$H-NMR (DMSO-$d_6$)δ: 0.86 (3H, t, J = 7.1Hz), 1.28-1.33 (4H, m), 1.72-1.77 (2H, m), 2.42 (3H, s), 2.77 (2H, t, J = 7.6Hz), 3.95 (2H, s), 4.42 (2H, s), 5.47 (2H, s), 6.96-7.29 (8H, m), 7.50-7.67 (4H, m)
IR (KBr) $cm^{-1}$: 2950, 2930, 1605, 1570, 1345
MS (FAB) m/z: 535 (M + 1)

Example 26: Preparation of 5-(3-hydroxymethylbenzyl)-6-methyl-2-pentyl-3-[2'-(1H-tetrazol-5-yl)biphenyl-4-yl]methylpyrimidin-4-one

The compound prepared in Reference Example 40 was treated in accordance with the method described in above-mentioned Example 1 to obtain the above-titled compound.
Yield: 37%,
Melting point: 100-102 ° C
$^1$H-NMR (DMSO-$d_6$)δ: 0.81 (3H, t, J = 7.1Hz), 1.15-1.32 (4H, m), 1.53-1.65 (2H, m), 2.28 (3H, s), 2.71 (2H, t, J = 7.7Hz), 3.85 (2H, s), 4.45 (2H, s), 5.32 (2H, s), 7.05-7.23 (8H, m), 7.49-7.69 (4H, m)
IR (KBr) $cm^{-1}$: 2960, 2930, 1700, 1655, 1540
MS (FAB) m/z: 535 (M + 1)

Examples 27 and 28

The compound prepared in Reference Example 41 was treated in accordance with the method described in above-mentioned Examples 1 and 2 to obtain the compounds described in the following Examples 27 and 28.

Example 27: Preparation of 6-ethyl-5-(3-hydroxymethylbenzyl)-2-methyl-3-[2'-(1H-tetrazol-5-yl)biphenyl-4-yl]methylpyrimidin-4-one

Yield: 47%,
Melting point: 103-105 ° C
$^1$H-NMR (DMSO-$d_6$)δ: 1.06 (3H, t, J = 7.6Hz), 2.39 (3H, s), 2.51-2.55 (2H, m), 3.86 (2H, s), 4.44 (2H, s), 5.28 (2H, s), 7.03-7.22 (8H, m), 7.51-7.69 (4H, m)

IR (KBr) $cm^{-1}$: 3420, 1650, 1605, 1540
MS (FAB) m/z: 493 (M+1)

Example 28: Preparation of 6-ethyl-5-(3-methoxymethylbenzyl)-2-methyl-3-[2'-(1H-tetrazol-5-yl)biphenyl-4-yl]methylpyrimidin-4-one

Yield: 45%,
Melting point: 88-90 °C
$^1$H-NMR (DMSO-$d_6$)δ: 1.04 (3H, t, J=7.5Hz), 2.38 (3H, s), 2.51-2.54 (2H, m), 3.25 (3H, s), 3.87 (2H, s), 4.34 (2H, s), 5.28 (2H, s), 7.09-7.25 (8H, m), 7.51-7.69 (4H, m)
IR (KBr) $cm^{-1}$: 1655, 1605, 1540, 1450
MS (FAB) m/z: 507 (M+1)

Example 29: Preparation of 2,6-diethyl-5-(3-hydroxymethylbenzyl)-4-[2'-(1H-tetrazol-5-yl)biphenyl-4-yl]-methoxypyrimidine

The compound prepared in Reference Example 42 was treated in accordance with the method described in above-mentioned Example 1 to obtain the above-titled compound.
Yield: 18%,
Melting point: 83-85 °C
$^1$H-NMR (DMSO-$d_6$)δ: 1.11 (3H, t, J=7.6Hz), 1.25 (3H, t, J=7.6Hz), 2.68 (2H, q, J=7.5Hz), 2.75 (2H, q, J=7.6Hz), 3.95 (2H, s), 4.41 (2H, s), 5.43 (2H, s), 6.93-7.28 (8H, m), 7.51-7.70 (4H, m)
IR (KBr) $cm^{-1}$: 3410, 1570, 1420, 1345
MS (FAB) m/z: 507 (M+1)

Examples 30 and 31

The compound prepared in Reference Example 43 was treated in accordance with the method described in above-mentioned Examples 1 and 2 to obtain the compounds described in the following Examples 30 and 31.

Example 30: Preparation of 2,6-diethyl-5-(3-hydroxymethylbenzyl)-3-[2'-(1H-tetrazol-5-yl)biphenyl-4-yl]-methoxypyrimidin-4-one

Yield: 33%,
Melting point: 96-98 °C
$^1$H-NMR (DMSO-$d_6$)δ: 1.06-1.14 (6H, m), 2.53 (2H, q, J=7.4Hz), 2.65 (2H, q, J=7.3Hz), 3.87 (2H, s), 4.44 (2H, s), 5.30 (2H, s), 7.08 (4H, s), 7.03-7.22 (4H, m), 7.50-7.69 (4H, m)
IR (KBr) $cm^{-1}$: 3425, 2985, 1650, 1540
MS (FAB) m/z: 507 (M+1)

Example 31: Preparation of 2,6-diethyl-5-(3-methoxymethylbenzyl)-3-[2'-(1H-tetrazol-5-yl)biphenyl-4-yl]-methylpyrimidin-4-one

Yield: 50%,
Melting point: 79-81 °C
$^1$H-NMR (DMSO-$d_6$)δ: 1.07 (3H, t, J=7.4Hz), 1.12 (3H, t, J=7.3Hz), 2.53 (2H, q, J=7.5Hz), 2.65 (2H, q, J=7.3Hz), 3.25 (3H, s), 3.88 (2H, s), 4.34 (2H, s), 5.30 (2H, s), 7.08 (4H, s), 7.09-7.25 (4H, m), 7.50-7.69 (4H, m)
IR (KBr) $cm^{-1}$: 3440, 2980, 1650, 1540
MS (FAB) m/z: 521 (M+1)

Example 32: Preparation of 6-ethyl-5-(3-hydroxymethylbenzyl)-2-propyl-4-[2'-(1H-tetrazol-5-yl)biphenyl-4-yl]-methoxypyrimidine

The compound prepared in Reference Example 44 was treated in accordance with the method described in above-mentioned Example 1 to obtain the above-titled compound.
Yield: 63%,

Melting point: 70-72 °C

$^1$H-NMR (DMSO-$d_6$)δ: 0.92 (3H, t, J = 7.4Hz), 1.07-1.21 (3H, m), 1.69-1.83 (2H, m), 2.63-2.77 (4H, m), 3.95 (2H, s), 4.41 (2H, s), 5.41 (2H, s), 6.92-7.27 (8H, m), 7.50-7.69 (4H,m)

IR (KBr) $cm^{-1}$: 3420, 1570, 1420, 1345

MS (FAB) m/z: 521 (M + 1)

Example 33: Preparation of 6-ethyl-5-(3-hydroxymethylbenzyl)-2-propyl-3-[2'-(1H-tetrazol-5-yl)biphenyl-4-yl]-methylpyrimidin-4-one

The compound prepared in Reference Example 45 was treated in accordance with the method described in above-mentioned Example 1 to obtain the above-titled compound.

Yield: 55%,

Melting point: 81-83 °C

$^1$H-NMR (DMSO-$d_6$)δ: 0.87 (3H, t, J = 7.4Hz), 1.08 (3H, t, J = 7.4Hz), 1.60-1.67 (2H, m), 2.48-2.63(4H, m), 3.87 (2H, s), 4.44 (2H, s), 5.30 (2H, s), 7.03-7.43 (8H, m), 7.50-7.70 (4H, m)

IR (KBr) $cm^{-1}$: 3420, 2970, 1650, 1540

MS (FAB) m/z: 521 (M + 1)

Examples 34 and 35

The compound prepared in Reference Example 46 was treated in accordance with the method described in above-mentioned Examples 1 and 2 to obtain the compounds described in the following Examples 34 and 35.

Example 34: Preparation of 2-butyl-6-ethyl-5-(3-hydroxymethylbenzyl)-4-[2'-(1H-tetrazol-5-yl)biphenyl-4-yl]-methoxypyrimidine

Yield: 45%,

Melting point: 63-65 °C

$^1$H-NMR (DMSO-$d_6$)δ: 0.89 (3H, t, J = 7.3Hz), 1.11 (3H, t, J = 7.4Hz), 1.30-1.38 (2H, m), 1.69-1.75 (2H, m), 2.67-2.77 (4H, m), 3.95 (2H, s), 4.41 (2H, s), 5.42 (2H, s), 6.92-7.27 (8H, m), 7.50-7.70 (4H, m)

IR (KBr) $cm^{-1}$: 2960, 1570, 1420, 1350

MS (FAB) m/z: 535 (M + 1)

Example 35: Preparation of 2-butyl-6-ethyl-5-(3-methoxymethylbenzyl)-4-[2'-(1H-tetrazol-5-yl)biphenyl-4-yl]-methoxypyrimidine

Yield: 42%,

Melting point: oil

$^1$H-NMR (DMSO-$d_6$)δ: 0.89 (3H, t, J = 7.3Hz), 1.10 (3H, t, J = 7.5Hz), 1.29-1.38 (2H, m), 1.66-1.78 (2H, m), 2.67-2.77 (4H, m), 3.21 (3H, s), 3.96 (2H, s), 4.31 (2H, s), 5.42 (2H, s), 6.97-7.26 (8H, m), 7.50-7.69 (4H, m)

IR (KBr) $cm^{-1}$: 1570, 1420, 1345, 1095

MS (FAB) m/z: 549 (M + 1)

Examples 36 and 37

The compound prepared in Reference Example 47 was treated in accordance with the method described in above-mentioned Examples 1 and 2 to obtain the compounds described in the following Examples 36 and 37.

Example 36: Preparation of 2-butyl-6-ethyl-5-(3-hydroxymethylbenzyl)-3-[2'-(1H-tetrazol-5-yl)biphenyl-4-yl]-methylpyrimidin-4-one

Yield: 36%,

Melting point: 81-83 °C

$^1$H-NMR (DMSO-$d_6$)δ: 0.81 (3H, t, J = 7.3Hz), 1.08 (3H, t, J = 7.5Hz), 1.24-1.32 (2H, m), 1.56-1.62 (2H, m), 2.53-2.65 (4H, m), 3.86 (2H, s), 4.44 (2H, s), 5.30 (2H, s), 7.08-7.35 (8H, m), 7.49-7.66 (4H, m)

IR (KBr) $cm^{-1}$: 2960, 2930, 1645, 1540
MS (FAB) m/z: 535 (M+1)

Example 37: Preparation of 2-butyl-6-ethyl-5-(3-methoxymethylbenzyl)-3-[2'-(1H-tetrazol-5-yl)biphenyl-4-yl]-methylpyrimidin-4-one

Yield: 40%,
Melting point: 64-66 ° C
$^1$H-NMR (DMSO-$d_6$)δ: 0.81 (3H, t, J=7.3Hz), 1.06 (3H, t, J=7.5Hz), 1.24-1.35 (2H, m), 1.53-1.64 (2H, m), 2.56-2.65 (4H, m), 3.25 (3H, s), 3.87 (2H, s), 4.34 (2H, s), 5.30 (2H, s), 7.08-7.40 (8H, m), 7.49-7.80 (4H, m)
IR (KBr) $cm^{-1}$: 2960, 2930, 1650, 1540
MS (FAB) m/z: 535 (M+1)

Example 38: Preparation of 5-(3-hydroxymethylbenzyl)-2-methyl-6-propyl-3-[2'-(1H-tetrazol-5-yl)biphenyl-4-yl]methylpyrimidin-4-one

The compound prepared in Reference Example 48 was treated in accordance with the method described in above-mentioned Example 1 to obtain the above-titled compound.
Yield: 72%,
Melting point: 89-90 ° C
$^1$H-NMR ($CDCl_3$)δ: 0.93 (3H, t, J=7.3Hz), 1.64 (2H, t, J=7.6Hz), 2.53 (3H, s), 2.74 (2H, t, J=7.6Hz), 3.95 (2H, s), 4.53 (2H, s), 5.37 (2H, s), 7.02-7.28 (8H, m), 7.41-7.91 (4H, m)
IR (KBr) $cm^{-1}$: 3420, 1570, 1425, 1345
MS (FAB) m/z: 507 (M+1)

Example 39: Preparation of 2-ethyl-5-(3-hydroxymethylbenzyl)-6-propyl-4-[2'-(1H-tetrazol-5-yl)biphenyl-4-yl]-methoxypyrimidine

The compound prepared in Reference Example 49 was treated in accordance with the method described in above-mentioned Example 1 to obtain the above-titled compound.
Yield: 50%,
Melting point: 78-80 ° C
$^1$H-NMR ($CDCl_3$)δ: 0.94 (3H, t, J=7.3Hz), 1.28 (3H, t, J=7.5Hz), 1.64 (2H, t, J=7.5Hz), 2.76-2.86 (4H, m), 3.95 (2H, s), 4.53 (2H, s), 5.41 (2H, s), 7.05-7.24 (4H, m), 7.34-7.60 (8H, m)
IR (KBr) $cm^{-1}$: 3420, 2970, 1570, 1420
MS (FAB) m/z: 521 (M+1)

Example 40: Preparation of 2-ethyl-5-(3-hydroxymethylbenzyl)-6-propyl-3-[2'-(1H-tetrazol-5-yl)biphenyl-4-yl]-methylpyrimidin-4-one

The compound prepared in Reference Example 50 was treated in accordance with the method described in above-mentioned Example 1 to obtain the above-titled compound.
Yield: 56%,
Melting point: 93-95 ° C
$^1$H-NMR ($CDCl_3$)δ: 0.93 (3H, t, J=7.4Hz), 1.25 (3H, t, J=7.3Hz), 1.56-1.70 (2H, m), 2.51-2.57 (2H, m), 2.65-2.74 (2H, m), 3.80 (2H, s), 4.50 (2H, s), 5.15 (2H, s), 7.03-7.24 (4H, m), 7.42-7.94 (8H, m)
IR (KBr) $cm^{-1}$: 3430, 2940, 1650, 1540
MS (FAB) m/z: 521 (M+1)

Example 41: Preparation of 5-(3-hydroxymethylbenzyl)-2,6-dipropyl-4-[2'-(1H-tetrazol-5-yl)biphenyl-4-yl]-methoxypyrimidine

The compound prepared in Reference Example 51 was treated in accordance with the method described in above-mentioned Example 1 to obtain the above-titled compound.
Yield: 50%,
Melting point: 75-77 ° C
$^1$H-NMR ($CDCl_3$)δ: 0.92-0.97 (6H, m), 1.60-1.82 (4H, m), 2.71-2.77 (4H, m), 3.94 (2H, s), 4.53 (2H, s),

5.38 (2H, s), 6.97-7.18 (8H, m), 7.37-7.88 (4H, m)

IR (KBr) $cm^{-1}$: 3420, 2960, 1570, 1420

MS (FAB) m/z: 535 (M + 1)

Example 42: Preparation of 5-(3-hydroxymethylbenzyl)-2,6-dipropyl-3-[2'-(1H-tetrazol-5-yl)biphenyl-4-yl]-methylpyrimidin-4-one

The compound prepared in Reference Example 52 was treated in accordance with the method described in above-mentioned Example 1 to obtain the above-titled compound.

Yield: 54%,

Melting point: 91-92 °C

$^1$H-NMR ($CDCl_3$)δ: 0.89-0.99 (6H, m), 1.55-1.63 (2H ,m), 1.66-1.82 (2H, m), 2.54 (2H, t, J = 7.7Hz), 2.65 (2H, t, J = 7.3Hz), 3.80 (2H, s), 4.50 (2H, s), 5.15 (2H, s), 6.97-7.19 (8H, m), 7.37-7.88 (4H, m)

IR (KBr) $cm^{-1}$: 3420, 2960, 1645, 1540

MS (FAB) m/z: 535 (M + 1)

Example 43: Preparation of 2-butyl-5-(3-hydroxymethylbenzyl)-6-propyl-4-[2'-(1H-tetrazol-5-yl)biphenyl-4-yl]-methoxypyrimidine

The compound prepared in Reference Example 53 was treated in accordance with the method described in above-mentioned Example 1 to obtain the above-titled compound.

Yield: 71%,

Melting point: 68-70 °C

$^1$H-NMR ($CDCl_3$)δ: 0.91 (3H, t, J = 7.3Hz), 0.95 (3H, t, J = 7.6Hz), 1.30-1.44 (2H, m), 1.58-1.80 (4H, m), 2.71-2.78 (4H, m), 3.90 (2H, s), 4.54 (2H, s), 5.37 (2H, s), 7.03-7.97 (12H, m)

IR (KBr) $cm^{-1}$: 3420, 2960, 1570, 1420

MS (FAB) m/z: 549 (M + 1)

Example 44: Preparation of 2-butyl-5-(3-hydroxymethylbenzyl)-6-propyl-3-[2'-(1H-tetrazol-5-yl)biphenyl-4-yl]-methylpyrimidin-4-one

The compound prepared in Reference Example 54 was treated in accordance with the method described in above-mentioned Example 1 to obtain the above-titled compound.

Yield: 58%,

Melting point: 71-73 °C

$^1$H-NMR ($CDCl_3$)δ: 0.93 (3H, t, J = 7.4Hz), 1.00 (3H, t, J = 7.4Hz), 1.32-1.41 (4H, m), 1.57-1.75 (4H, m), 2.51-2.68 (4H, m), 3.80 (2H, s), 4.51 (2H, s), 5.16 (2H, s), 7.01-7.89 (12H, m)

IR (KBr) $cm^{-1}$: 3420, 2960, 1645, 1540

MS (FAB) m/z: 549 (M + 1)

Example 45: Preparation of 6-ethyl-5-(4-hydroxymethylbenzyl)-2-methyl-4-[2'-(1H-tetrazol-5-yl)biphenyl-4-yl]methoxypyrimidine

The compound prepared in Reference Example 55 was treated in accordance with the method described in above-mentioned Example 1 to obtain the above-titled compound.

Yield: 39%,

Melting point: 103-105 °C

$^1$H-NMR (DMSO-$d_6$)δ: 1.09 (3H, t, J = 7.6Hz), 2.49 (3H, s), 2.66 (2H, q, J = 7.6Hz), 3.93 (2H, s), 4.43 (2H, s), 5.40 (2H, s), 7.01-7.08 (4H, m), 7.16-7.26 (4H, m), 7.51-7.68 (4H, m)

IR (KBr) $cm^{-1}$: 3410, 1570, 1420, 1345

MS (FAB) m/z: 493 (M + 1)

Example 46: Preparation of 6-ethyl-5-(4-hydroxymethylbenzyl)-2-methyl-3-[2'-(1H-tetrazol-5-yl)biphenyl-4-yl]methylpyrimidin-4-one

The compound prepared in Reference Example 56 was treated in accordance with the method described in above-mentioned Example 1 to obtain the above-titled compound.

Yield: 27%,

Melting point: 121-123 °C

$^1$H-NMR (DMSO-$d_6$)δ: 1.05 (3H, t, J = 7.5Hz), 2.38 (3H, s), 2.50(2H, q, J = 7.5Hz), 3.85 (2H, s), 4.44 (2H, s), 5.27 (2H, s), 7.10-7.21 (8H, m), 7.51-7.69 (4H, m)

IR (KBr) $cm^{-1}$: 3430, 1650, 1540, 1425

MS (FAB) m/z: 493 (M + 1)

Example 47: Preparation of 6-ethyl-5-(3-hydroxymethylbenzyl)-2-methyl-4-[2'-(1H-tetrazol-5-yl)biphenyl-4-yl]methoxypyrimidine

The compound prepared in Reference Example 57 was treated in accordance with the method described in above-mentioned Example 1 to obtain the above-titled compound.

Yield: 37%,

Melting point: 99-101 °C

$^1$H-NMR (DMSO-$d_6$)δ: 1.11 (3H, t, J = 7.6Hz), 2.50 (3H, s), 2.68 (2H, q, J = 7.6Hz), 3.95 (2H, s), 4.41 (2H, s), 5.42 (2H, s), 6.93-7.35 (8H, m), 7.51-7.58 (2H, m), 7.64-7.69 (2H, m)

IR (KBr) $cm^{-1}$: 3600-3300, 1570, 1425, 1345

MS (FAB) m/z: 493 (M + 1)

Example 48: Preparation of 5-(3- acetoxymethylbenzyl)-6-methyl-2-propyl-3-[2'-(triphenylmethyltetrazol-5-yl)biphenyl-4-yl]methylpyrimidin-4-one

(a) Preparation of 5-(3-hydroxymethylbenzyl)-6-methyl-2-propyl-3-[2'-(triphenylmethyltetrazol-5-yl)biphenyl-4-yl]methylpyrimidin-4-one

The compound prepared in Reference Example 36 was treated in accordance with the method described in above-mentioned Example 1 (a) to obtain the above-titled compound.

Yield: 70%,

Melting point: amorphous

$^1$H-NMR ($CDCl_3$)δ: 0.88 (3H, t, J = 7.3Hz), 1.61-1.69 (2H, m), 2.32 (3H, s), 2.50 (2H, t, J = 7.7Hz), 3.95 (2H, s), 4.62 (2H, s), 5.20 (2H, s), 6.91-6.95 (8H, m), 7.09 (2H, d, J = 7.8Hz), 7.16-7.35 (14H, m), 7.42-7.51 (2H, m), 7.89-7.92 (1H, m)

IR (KBr) $cm^{-1}$: 3600-3300, 1655, 1540, 1445

MS (FAB) m/z: 749 (M + 1)

(b) Preparation of 5-(3- acetoxymethylbenzyl)-6-methyl-2-propyl-3-[2'-(triphenylmethyltetrazol-5-yl)biphenyl-4-yl]methylpyrimidin-4-one

The compound (7.6 g) prepared in Example 48 (a) was dissolved in dichloromethane (40 ml). Triethylamine (1.8 ml) was added to the solution. After acetyl chloride (0.8 ml) was added dropwise under stirring while cooling on ice, the mixture was stirred at room temperature overnight. After the reaction was completed, the reaction mixture was washed with water and saturated saline and dried over anhydrous magnesium sulfate. The solvent was evaporated under reduced pressure and the resulting crystals were recrystallized from ethanol/water to obtain the above-captioned compound (5.6 g).

Yield: 69%,

Melting point: 135-137 °C (decomposition)

$^1$H-NMR ($CDCl_3$)δ: 0.88 (3H, t, J = 7.3Hz), 1.58-1.72 (2H, m), 2.06 (3H, s), 2.33 (3H, s), 2.51 (2H, t, J = 7.7Hz), 3.95 (2H, s), 5.06 (2H, s), 5.20 (2H, s), 6.90-6.95 (6H, m), 7.10 (2H, d, J = 8.1Hz), 7.16-7.35 (16H, m), 7.41-7.51 (2H, m), 7.90-7.93 (1H, m)

IR (KBr) $cm^{-1}$: 3700-3300, 1740, 1655, 1545, 1445

MS (FAB) m/z: 791 (M + 1)

Example 49: Preparation of 3-(2'-cyanobiphenyl-4-yl)methyl-5-(3-hydroxymethylbenzyl)-6-methyl-2-propylpyrimidin-4-one

(a) Preparation of 3-(2'-cyanobiphenyl-4-yl)methyl-5-(3-methoxycarbonylbenzyl)-6-methyl-2-propylpyrimidin-4-one

To a suspension of cesium carbonate (97.8 g) in dioxane (370 ml), the compound (45.0 g) prepared in Reference Example 9 was added. The mixture was stirred at room temperature for 20 minutes. In dioxane (740 ml), 4'-bromomethyl-2-cyanobiphenyl (40.6 g) was dissolved and the resulting solution was added dropwise to the mixture. The whole was stirred at room temperature overnight. Further, the reaction mixture was heated under reflux for 3 hours. The solvent was evaporated under reduced pressure. Water was added to dissolve the resulting residue. The precipitated crystals were collected by filtration and recrystallized from ethyl acetate to obtain the above-captioned compound (56.8 g).

Yield: 85%,

Melting point: 143-144 °C

$^1$H-NMR ($CDCl_3$)δ: 0.98 (3H, t, J = 7.3Hz), 1.70-1.78 (2H, m), 2.34 (3H, s), 2.65 (2H, t, J = 7.8Hz), 3.89 (2H, s), 3.99 (2H, s), 5.37 (2H, s), 7.25-7.37 (3H, m), 7.41-7.55 (5H, m), 7.61-7.67 (1H, m), 7.76 (1H, d, J = 7.6Hz), 7.86 (1H, d, J = 7.6Hz), 7.93 (1H, s)

IR (KBr) $cm^{-1}$: 2225, 1725, 1660, 1540, 1285

MS (FAB) m/z: 492 (M + 1)

(b) Preparation of 5-(3-carboxybenzyl)-3-(2'-cyanobiphenyl-4-yl)methyl-6-methyl-2-propylpyrimidin-4-one

The compound (54.1 g) prepared in Example 49 (a) was dissolved in methanol (500 ml). To the solution, 2N NaOH (150 ml) was added. The whole was stirred at 70 °C for 1 hour and concentrated under reduced pressure. Water (200 ml) was added to dissolved the resulting residue, and the solution was washed with ether (300 ml) twice. The pH of the resulting solution was adjusted to 4 with concentrated hydrochloric acid. The precipitated crystals were collected by filtration and recrystallized from ethanol to obtain the above-titled compound (38.0 g).

Yield: 72%,

Melting point: 169-170 °C

$^1$H-NMR ($CDCl_3$)δ: 0.98 (3H, t, J = 7.3Hz), 1.67-1.79 (2H, m), 2.34 (3H, s), 2.67(2H, t, J = 7.8Hz), 4.01 (2H, s), 5.39 (2H, s), 7.26-7.61 (9H, m), 7.73 (1H, dd, J = 7.8Hz, 1.2Hz), 7.88 (1H, d, J = 7.8Hz), 7.95(1H, s)

IR (KBr) $cm^{-1}$: 2225, 1710, 1655, 1540, 1445

MS (FAB) m/z: 478 (M + 1)

(c) Preparation of 3-(2'-cyanobiphenyl-4-yl)methyl-5-(3-hydroxymethylbenzyl)-6-methyl-2-propylpyrimidin-4-one

The compound (30 g) prepared in Example 49 (b) was dissolved in dry THF (300 ml). Under stirring at -10 °C, N-methylmorpholine (6.92 ml) and ethyl chloroformate (6.58 ml) were added to the solution. After the whole was further stirred for 10 minutes, sodium borohydride (7.18 g) was added. Under stirring while cooling on ice, methanol (600 ml) was added dropwise slowly. After the whole was stirred at room temperature for 30 minutes, the solvent was evaporated under reduced pressure. Water (500 ml) was added to dissolve the resulting residue, and the solution was extracted with ethyl acetate (500 ml) twice. The ethyl acetate extract layer was washed with saturated saline and dried over anhydrous sodium sulfate. The solvent was evaporated under reduced pressure and the resulting crystalls were recrystallized from ethanol/water to obtain the above-titled compound (25.4 g).

Yield: 87%,

Melting point: 111-112 °C

$^1$H-NMR ($CDCl_3$)δ: 0.95 (3H, t, J = 7.4Hz), 1.66-1.80(2H, m), 2.34 (3H, s), 2.64(2H, t, J = 7.8Hz), 3.94 (2H, s), 4.62 (2H, s), 5.36 (2H, s), 7.16-7.27 (6H, m), 7.41-7.54 (4H, m), 7.64 (1H, dt, J = 7.7Hz, 1.2Hz), 7.75 (1H, d, J = 7.8Hz)

IR (KBr) $cm^{-1}$: 2225, 1655, 1540, 1445

MS (FAB) m/z: 464 (M + 1)

Example 50: Preparation of 4-(2'-cyanobiphenyl-4-yl)methoxy-2,6-diethyl-5-(3-hydroxymethylbenzyl)-pyrimidine

(a) Preparation of 4-(2'-cyanobiphenyl-4-yl)methoxy-2,6-diethyl-5-(3-methoxycarbonylbenzyl)pyrimidine

The compound prepared in Reference Example 13 was treated with 4'-bromomethyl-2-cyanobiphenyl in accordance with the method described in above-mentioned Reference Example 21 (a) to obtain the above-titled compound.

Yield: 20%,

Melting point: oil

$^1$H-NMR ($CDCl_3$)δ: 1.20 (3H, t, J=7.6Hz), 1.36 (3H, t, J=7.6Hz), 2.75 (2H, q, J=7.6Hz), 2.87 (2H, q, J=7.6Hz), 3.87 (3H, s), 4.05 (2H, s), 5.49 (2H, s), 7.28-7.51 (8H, m), 7.60-7.66 (1H, m), 7.74-7.77 (1H, m), 7.85-7.88 (2H, m)

IR (KBr) $cm^{-1}$: 2225, 1720, 1575, 1560, 1285

MS (FAB) m/z: 492 (M+1)

(b) Preparation of 4-(2'-cyanobiphenyl-4-yl)methoxy-2,6-diethyl-5-(3-hydroxymethylbenzyl)pyrimidine

The compound prepated in Example 50 (a) was treated in accordance with the method described in above-mentioned Example 49 (b) and (c) to obtain the above-titled compound.

Yield: 61%,

Melting point: 111-112°C

$^1$H-NMR ($CDCl_3$)δ: 1.20 (3H, t, J=7.6Hz), 1.36 (3H, t, J=7.6Hz), 2.75 (2H, q, J=7.6Hz), 2.87 (2H, t, J=7.6Hz), 4.03 (2H, s), 4.59 (2H, s), 5.49 (2H, s), 7.20-7.52 (8H, m), 7.58-7.92 (4H, m)

IR (KBr) $cm^{-1}$: 2225, 1570, 1420

MS (FAB) m/z: 464 (M+1)

Formulation Example 1

| | |
|---|---|
| Compound of Example 3 | 20 g |
| Lactose | 100 g |
| Corn starch | 36 g |
| Microcrystalline cellulose | 30 g |
| Calcium carboxylmethylcellulose | 10 g |
| Magnesium stearate | 4 g |

The components of the above composition were homogeneously mixed to prepare tablets of 200 mg per tablet.

Formulation Example 2

| | |
|---|---|
| Compound of Example 19 | 20 g |
| Lactose | 315 g |
| Corn starch | 125 g |
| Microcrystalline cellulose | 25 g |

The components of the above composition were homogeneously mixed, and then granulated to prepare granules.

Formulation Example 3

| | |
|---|---|
| Compound of Example 20 | 20 g |
| Lactose | 100 g |
| Microcrystalline cellulose | 70 g |
| Magnesium stearate | 10 g |

The components of the above composition were homogeneously mixed, then granulated and encapsulated into gelatine capsules to prepare capsules of 200 mg content per capsule.

Formulation Example 4

| | |
|---|---|
| Sodium salt of compound of Example 23 | 5 g |
| Glucose | 50 g |
| Benzyl alcohol | 10 g |

The components of the above composition were dissolved in distilled water for injections so that a total volume became 1000 ml. The solution was poured into ampules to prepare an injection of 1 ml per ampule.

Pharmacological Example 1: Angiotension II receptor binding

The specific affinity of the compound of the present invention to an angiotensin II receptor was evaluated by substitution of radioactive ligands bound to an angiotensin II receptor prepared from rat liver.

(1) Preparation of angiotensin II receptor

The liver was obtained from 5-week-old Wistar male rat (Japan Charles River Inc.) and homogenized in the homogenizing buffer to prepare 10% concentration preparation. The homogenate was centrifuged at 3000 x g for 15 minutes and the supernatant was collected. The collected supernatant was centrifuged at 50000 x g for 30 minutes to obtain pellets. The homogenizing buffer (20 ml) was added to resuspend the resulting pellets and the angiotensin II receptor was prepared. The receptor was frozen at -80 °C until use.

Homogenizing Buffer
10 mM HEPES (pH 7.4) (*)
0.2% bovine serum albumin
10 µM pepstatin A
10 µM bestatin
10 µM leupeptin
10 µM captopryl
100 µM phenylmethanesulfonyl fluoride

(2) Receptor binding assay

The above receptor was thawed, and diluted to 250 µg/ml protein with the assay buffer. Then, the receptor, 0.5nM [$^3$H]-angiotensin II and the compound which was diluted to the various concentrations were incubated at 25 °C for 1 hour. The reaction mixture was filtered under suction through a glass fiber filter (Whatman GF/B filter) to commence the reaction and at the same time separate the [$^3$H]-angiotensin II bound to the receptor and the free [$^3$H]-angiotensin II. After the filter was rinsed with a washing buffer (10 mM HEPES, pH 7.4), the radioactivity absorbed to the filter was measured by a liquid scintillation counter (Beckman). The non-specific bindings were measured after adding $10^{-5}$ M non-labeled angiotensin II to the reaction.

Assay Buffer
10 mM HEPES (pH 7.4)

(*) HEPES = N-(2-hydroxyethyl)piperadine-N′-2-ethansulfonate

0.2% bovine serum albumin
10 mM $MgCl_2$

(3) Calculation of $IC_{50}$ value

The $IC_{50}$ value was calculated by the formula:

$$Y = T - S \times D / (D + K)$$

(R.J. Bruns, et. al., Molecular Pharmacology, 29: 331-346, 1986). In the formula, Y is the bound radioactivity in the presence of the compound to be tested, T is the bound radioactivity in the absence of the compound to be tested, S is the specifically bound radioactivity in the absence of the compound to be tested, D is the concentration of the compound to be tested, and K is the $IC_{50}$ value of the compound to be tested. The test results are shown in Table 1.

Table 1

| Compound to be tested | $IC_{50}$ (M) |
|---|---|
| Compound of Example 3 | $4.3 \times 10^{-8}$ |
| Compound of Example 4 | $1.1 \times 10^{-8}$ |
| Compound of Example 6 | $2.9 \times 10^{-8}$ |
| Compound of Example 7 | $1.6 \times 10^{-8}$ |
| Compound of Example 9 | $1.6 \times 10^{-8}$ |
| Compound of Example 13 | $1.4 \times 10^{-8}$ |
| Compound of Example 15 | $1.2 \times 10^{-8}$ |
| Compound of Example 16 | $8.1 \times 10^{-9}$ |
| Compound of Example 17 | $9.6 \times 10^{-9}$ |
| Compound of Example 18 | $1.2 \times 10^{-8}$ |
| Compound of Example 19 | $3.8 \times 10^{-9}$ |
| Compound of Example 20 | $6.6 \times 10^{-9}$ |
| Compound of Example 22 | $3.0 \times 10^{-8}$ |
| Compound of Example 23 | $8.3 \times 10^{-9}$ |
| Compound of Example 24 | $1.8 \times 10^{-8}$ |
| Compound of Example 27 | $1.8 \times 10^{-8}$ |
| Compound of Example 29 | $2.7 \times 10^{-8}$ |
| Compound of Example 30 | $9.6 \times 10^{-9}$ |
| Compound of Example 31 | $1.2 \times 10^{-8}$ |
| Compound of Example 34 | $2.5 \times 10^{-8}$ |
| Compound of Example 38 | $2.2 \times 10^{-8}$ |
| Compound of Example 40 | $2.4 \times 10^{-8}$ |
| Compound of Example 42 | $2.3 \times 10^{-8}$ |
| Compound of Example 47 | $9.9 \times 10^{-9}$ |

Pharmacological Example 2: Inhibitory activity of vasoconstriction

The applicability of the compound of the present invention was confirmed by the function to inhibit constriction of rat aorta induced by angiotensin II.

(1) Measurement of vasoconstriction using Magnus apparatus

Stetharteries of Wistar male rats having body weights of 150 to 250 g (Japan Charles River Inc.) were excised. After the attached connective tissue was removed, the cavities of the veins were rubbed by yarn to remove the endothelial cells and prepare ring samples. The samples were suspended in a 5ml organ bath which was filled with Krebs-Henseleit solution (unit mM; NaCl = 118, KCl = 4.7, $CaCl_2$ = 2.55, $MgSO_4$ = 1.18, $KH_2PO_4$ = 1.18, $NaHCO_3$ = 24.88, glucose = 11.1) and held at 37°C and bubbled with 95% $O_2$-5% $CO_2$. After an initial tension of 1.5 g was applied and preincubation was carried out for 60 minutes, the

compounds to be tested ($3 \times 10^{-8}$ or $1 \times 10^{-8}$) were administered. After 15 minutes, $3 \times 10^{-8}$ angiotensin II was accumulatively added and the reaction of constriction was measured. The rate of inhibition was evaluated by comparing the reaction of constriction, which had been previously measured, in the case of administration of $3 \times 10^{-8}$ angiotensin II in the absence of the compounds to be tested.

The constriction of the samples was ionotropicaly measured using an isometric transducer (UL-10GR: Minebea) and amplifier (6M92: NEC SANEI) and recorded by a recticorder (RECTI-HORIZ-8K: NEC SANEI). The test results are shown in Table 2.

Table 2

| Compound to be tested | Rate of inhibition (%) | |
|---|---|---|
| | $1 \times 10^{-8}$ M | $3 \times 10^{-8}$ M |
| Compound of Example 3 | 78 | 98 |
| Compound of Example 4 | 36 | 94 |
| Compound of Example 19 | 100 | 100 |
| Compound of Example 20 | 100 | 100 |
| Compound of Example 23 | 95 | 100 |
| Comparative compound A [a)] | 8 | 37 |
| Comparative compound B [b)] | 5 | 32 |

a):
2-butyl-6-methyl-3-[2'-(1H-tetrazol-5-yl)biphenyl-4-yl]methylpyrimidin-4-one: compound described in European Publication No. 0407342

b):
5-benzyl-2-butyl-6-methyl-3-[2'-(1H-tetrazol-5-yl)biphenyl-4-yl]methylpyrimidin-4-one

As clear from Table 2, the compound of the general formula (I) of the present invention is superior in activity of inhibiting vasoconstriction, in comparison with the compound described in European Publication No. 0407342 and the pyrimidine derivative having a benzyl group at 5-position used as the comparative compounds.

Toxicity Example

The compounds of Examples 3, 19, 20, or 23 were intraperitoneally administered to 4- to 5-week-old ICR male mice in an amount of 500 mg/kg. No deaths were observed in any of the groups.

The compounds of Examples 15, 19, or 47 were orally administered to 6-week-old ICR male and female mice in an amount of 1000 mg/kg. No deaths were observed in any of the groups.

INDUSTRIAL APPLICABILITY

As explained above, the compound of the general formula (I) of the present invention exhibits a superior angiotensin II antagonism and is very safe, and thus is useful as an effective ingredient of a pharmaceutical composition, in particular, a superior agent for the cardiovascular system. Accordingly, the compound of the present invention is useful as an agent for the treatment of various cardiovascular diseases, for example, hypertension, cardiac disease, cerebral infarction, and arteriosclerosis.

As above, the present invention was explained with reference to particular embodiments, but modifications and improvements obvious to those skilled in the art are included in the scope of the present invention.

The structural formulas of the compounds described in Example 1 to Example 50 are shown hereinafter. In the following structural formulas, Me is a methyl group, Et is an ethyl group, Pr is a propyl group, Bu is a butyl group, Pen is a pentyl group, Ac is an acetyl group, and Ph is a phenyl group.

Example 1

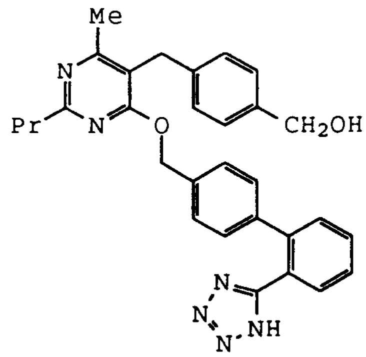

Example 2

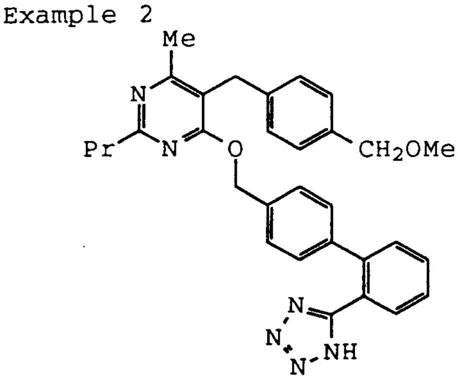

Example 3

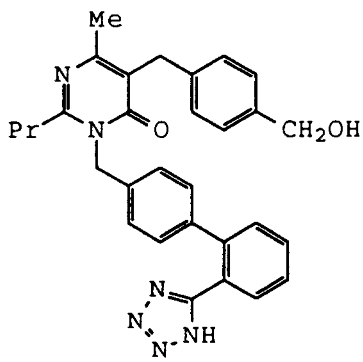

Example 4

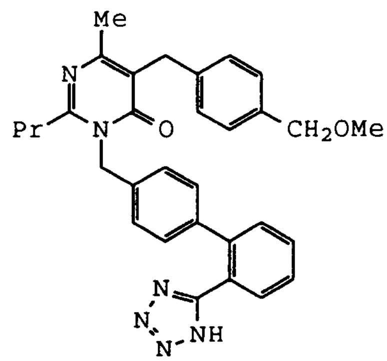

Example 5

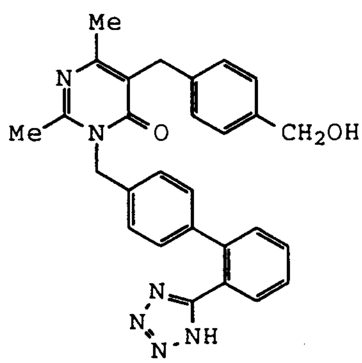

Example 6

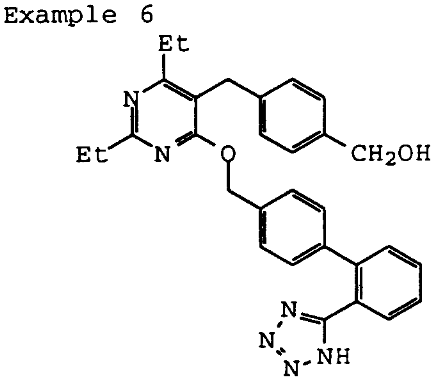

Example 7

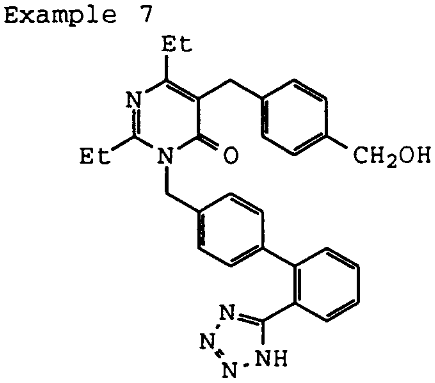

Example 8

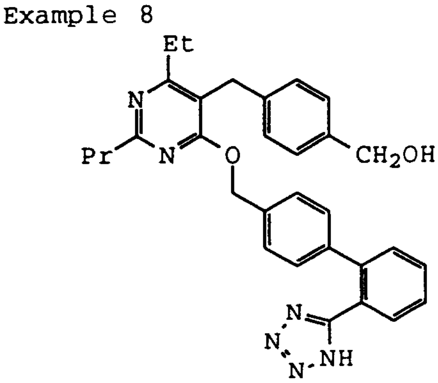

Example 9

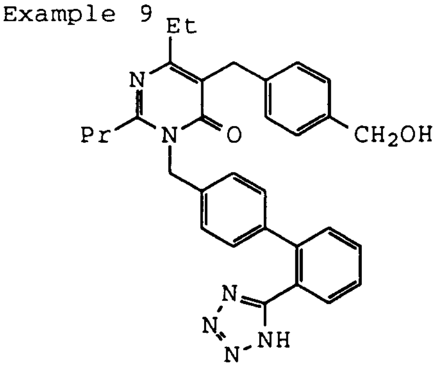

Example 10

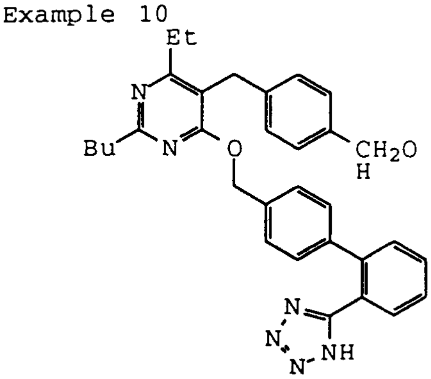

Example 11

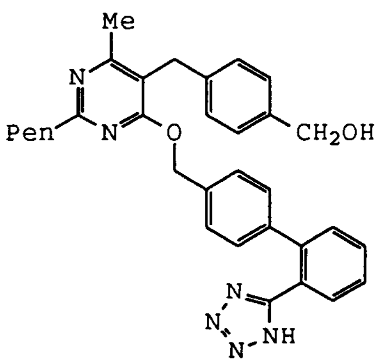

Example 12

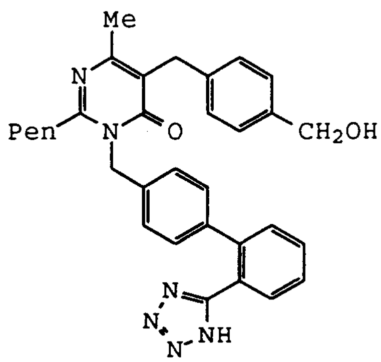

Example 13

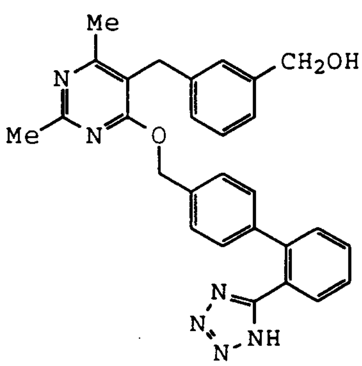

Example 14

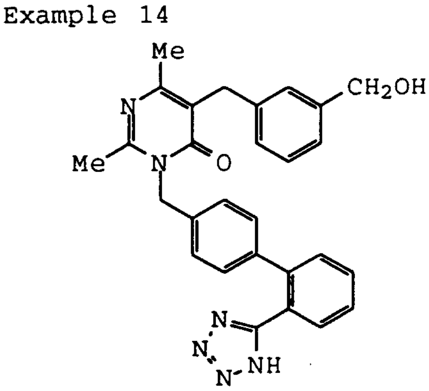

Example 15

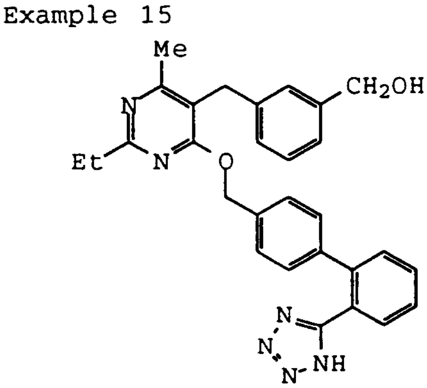

Example 16

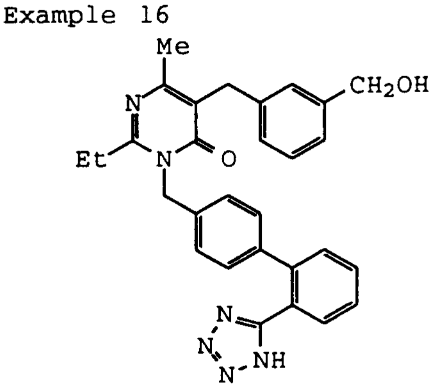

Example 17

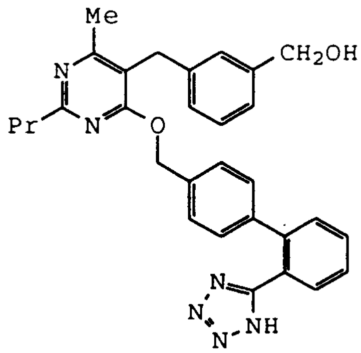

Example 18

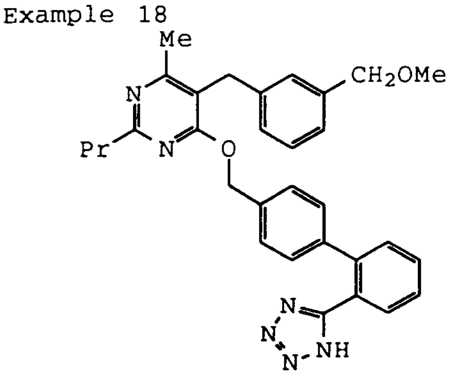

Example 19

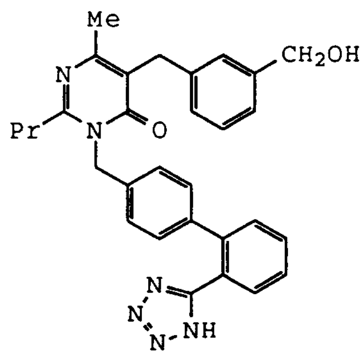

Example 20

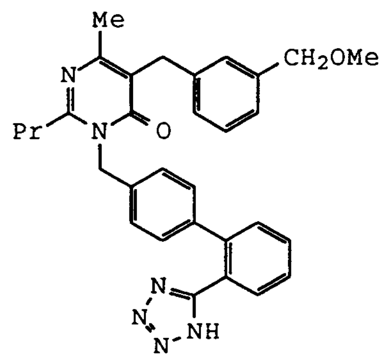

Example 21

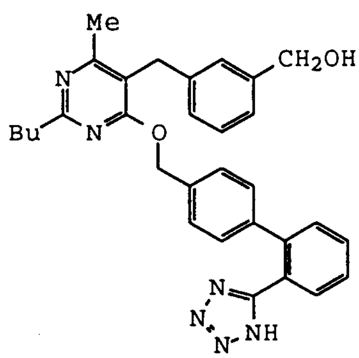

Example 22

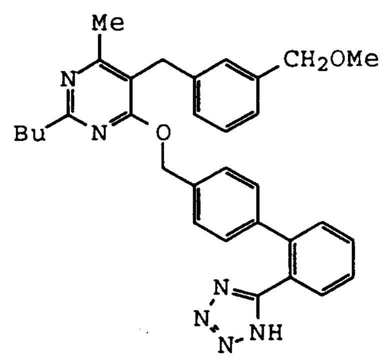

Example 23

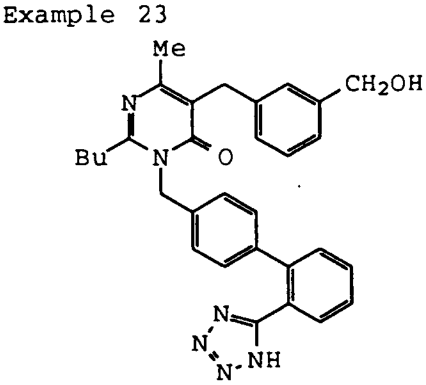

Example 24

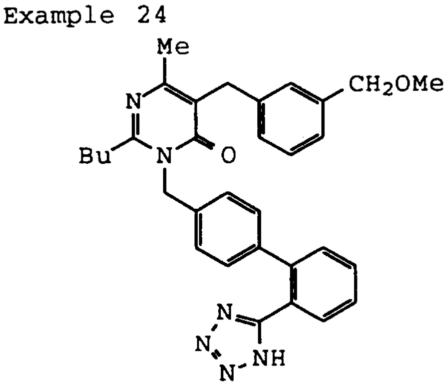

Example 25

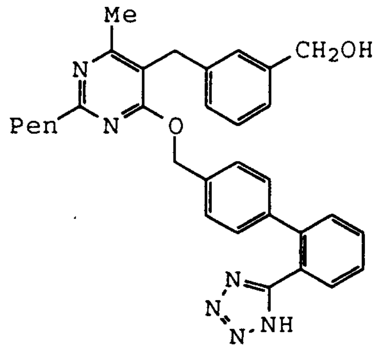

Example 26

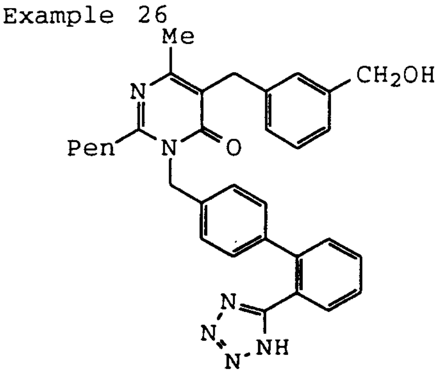

Example 27

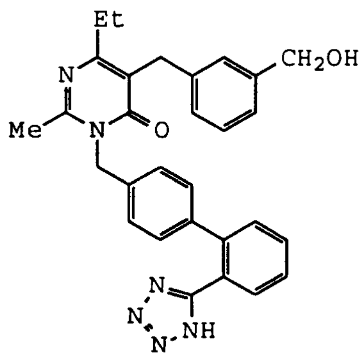

Example 28

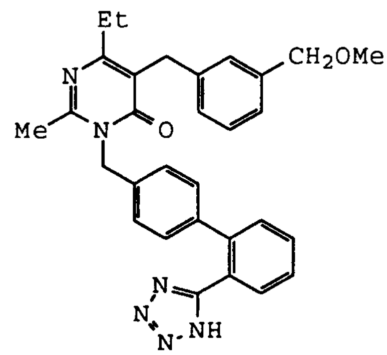

Example 29

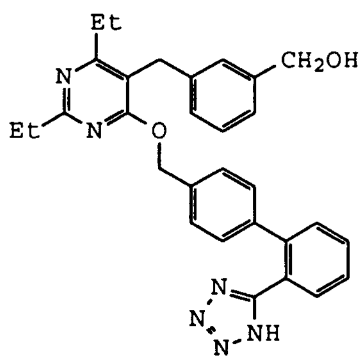

Example 30

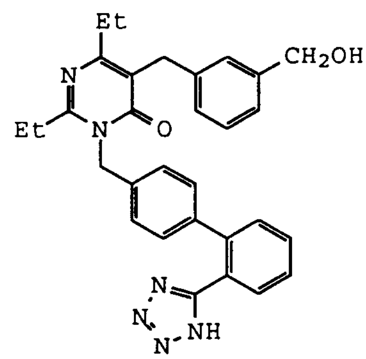

Example 31

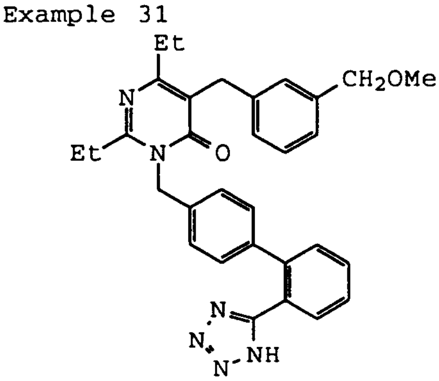

Example 32

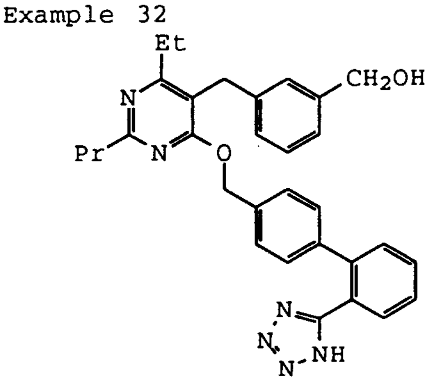

Example 33

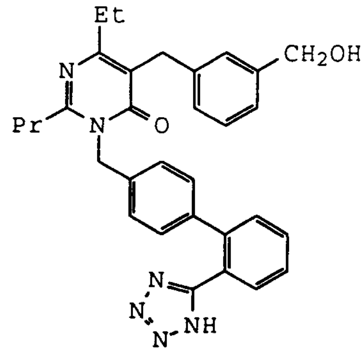

Example 34

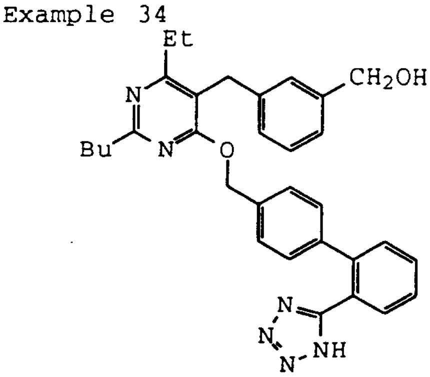

Example 35

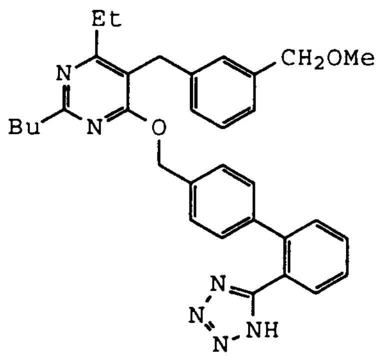

Example 36

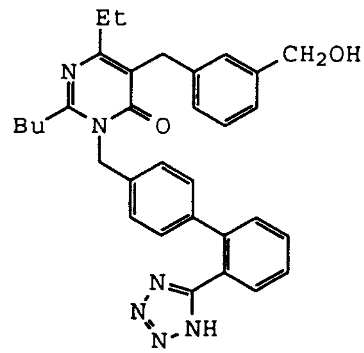

Example 37

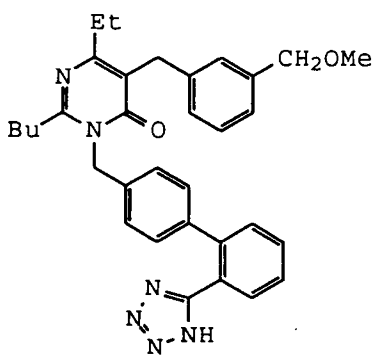

Example 38

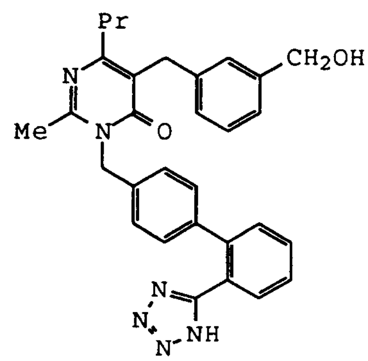

Example 39

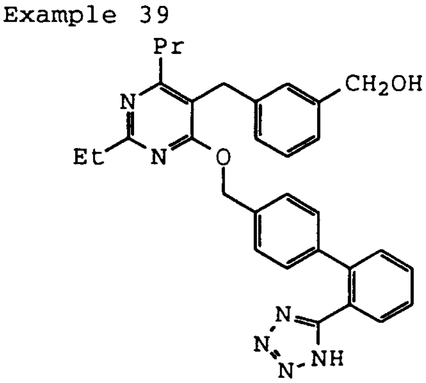

Example 40

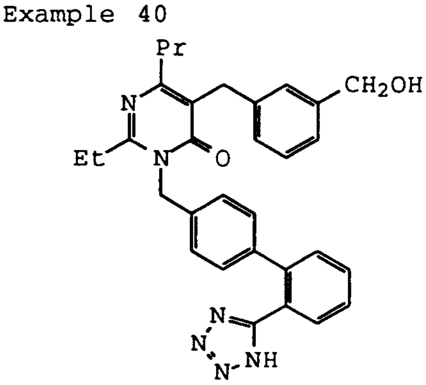

Example 41

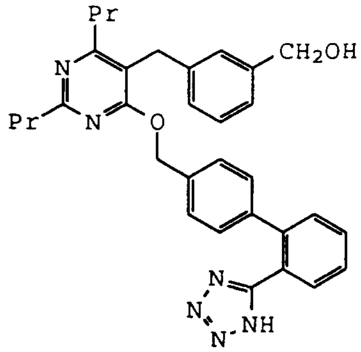

Example 42

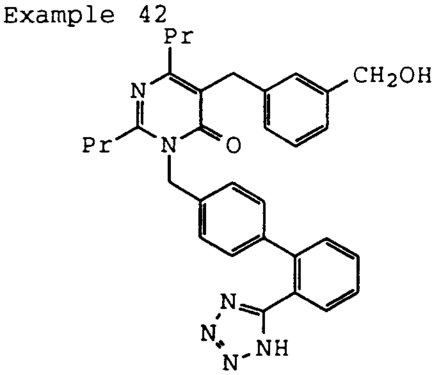

Example 43

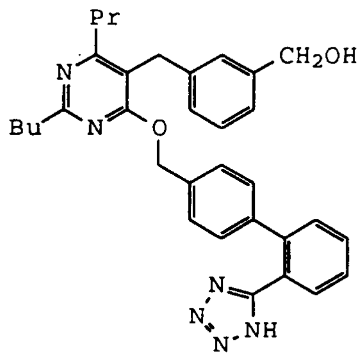

Example 44

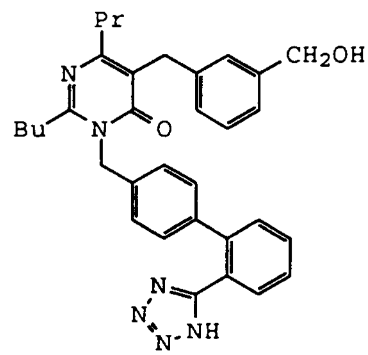

Example 45

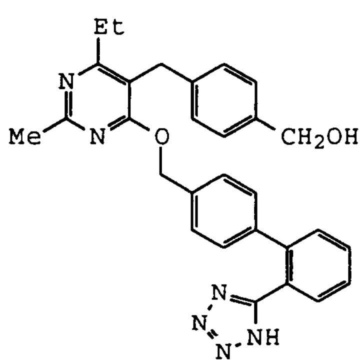

Example 46

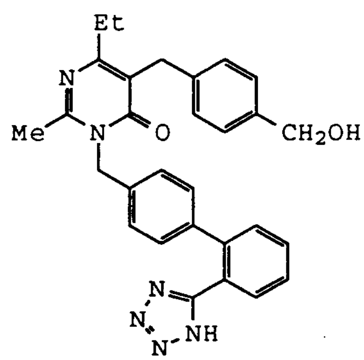

Example 47

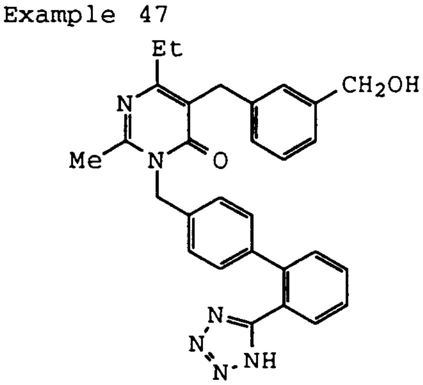

Example 48

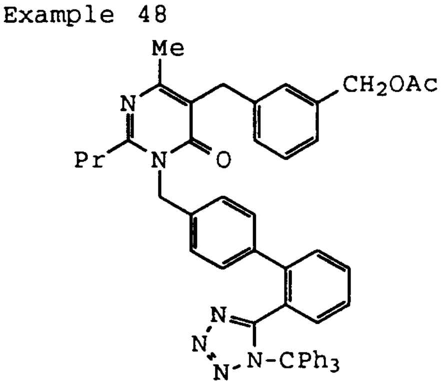

Example 49

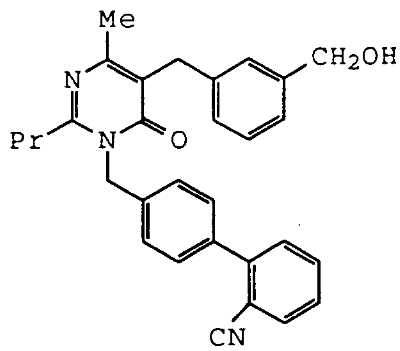

Example 50

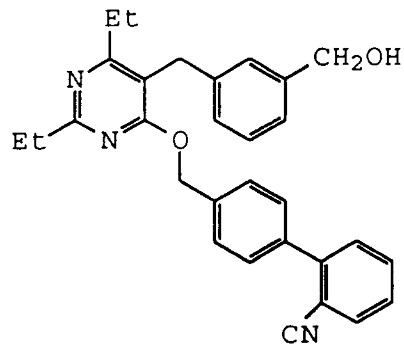

Reference Example 1

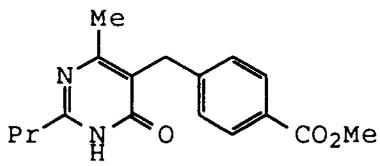

Reference Example 2

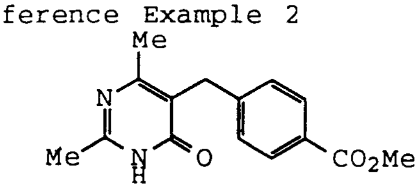

Reference Example 3

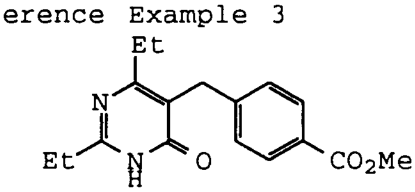

Reference Example 4

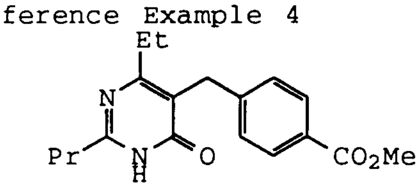

Reference Example 5

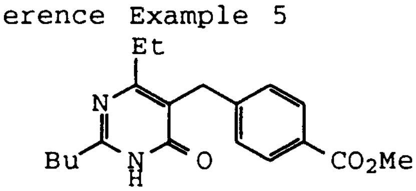

Reference Example 6

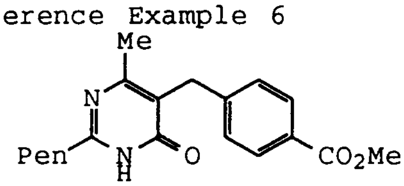

Reference Example 7

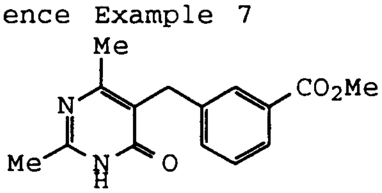

Reference Example 8

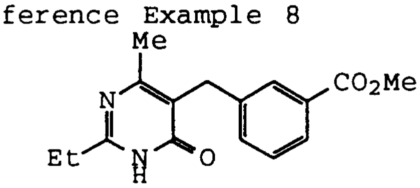

Reference Example 9

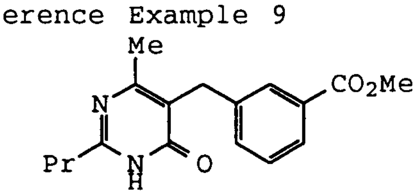

Reference Example 10

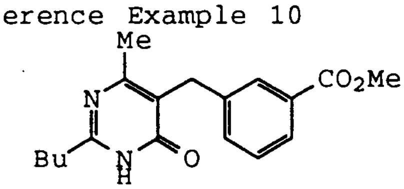

Reference Example 11

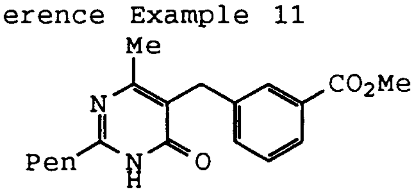

Reference Example 12

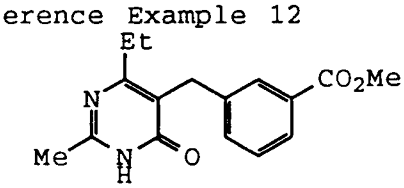

Reference Example 13

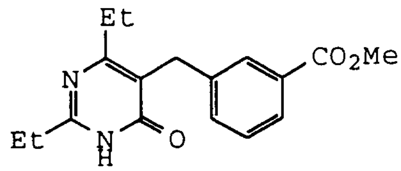

Reference Example 14

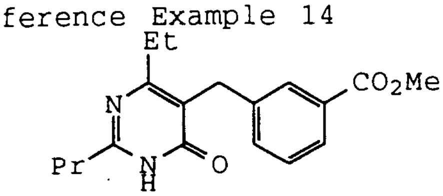

Reference Example 15

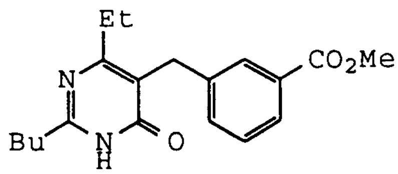

Reference Example 16

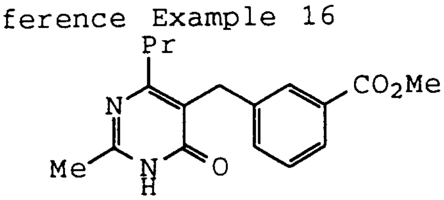

Reference Example 17

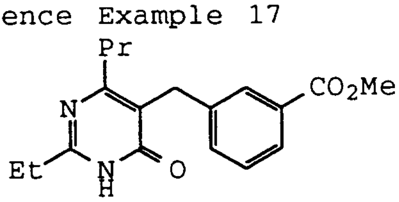

Reference Example 18

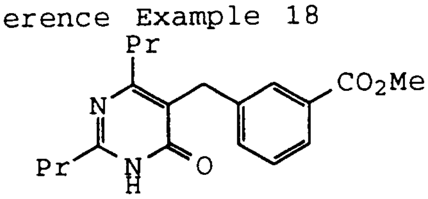

Reference Example 19

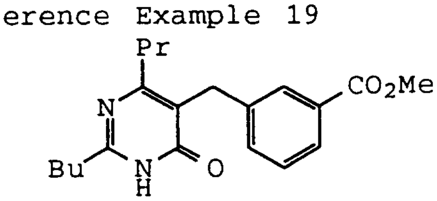

Reference Example 20

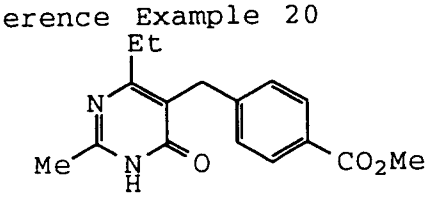

Reference Example 21

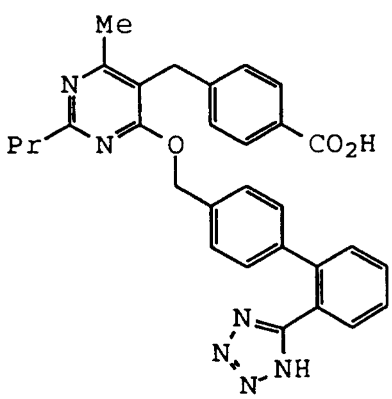

Reference Example 22

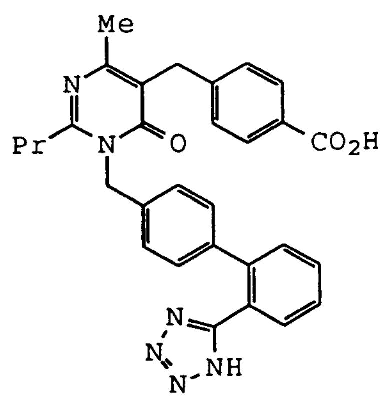

Reference Example 23

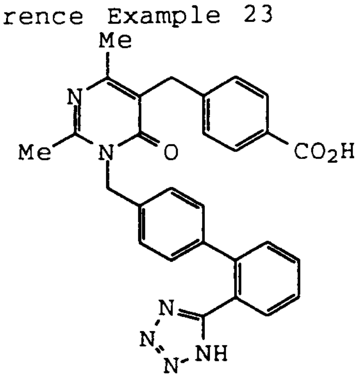

Reference Example 24

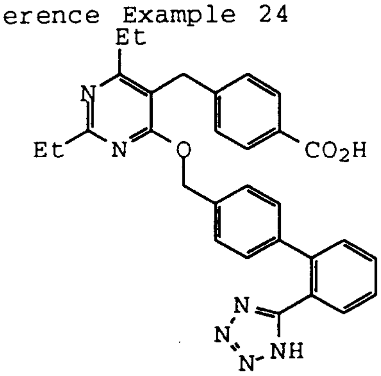

Reference Example 25

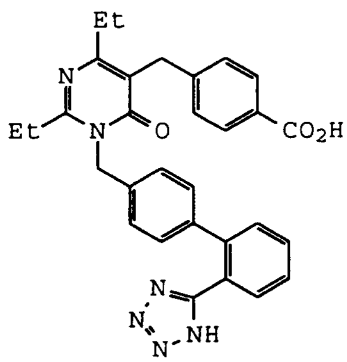

Reference Example 26

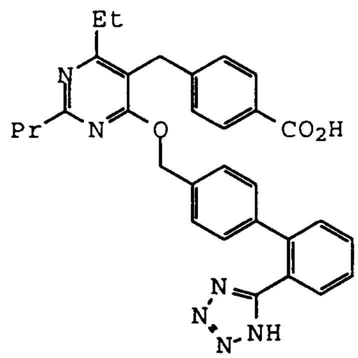

Reference Example 27

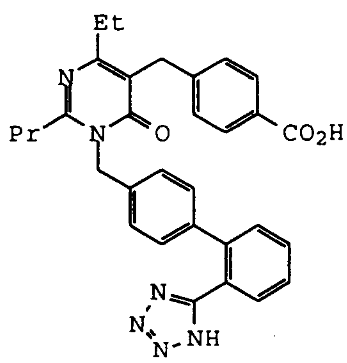

Reference Example 28

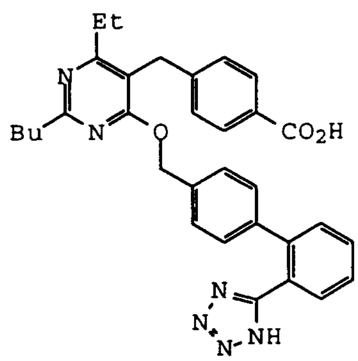

Reference Example 29

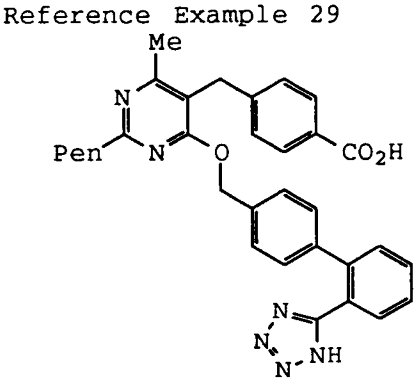

Reference Example 30

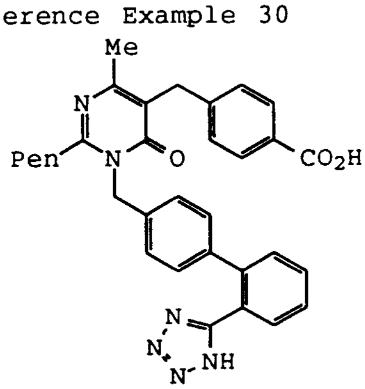

Reference Example 31

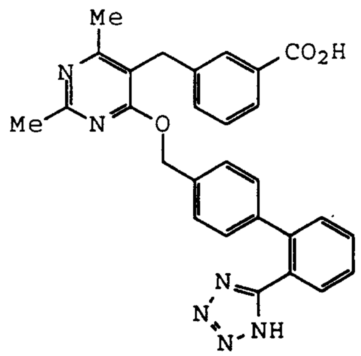

Reference Example 32

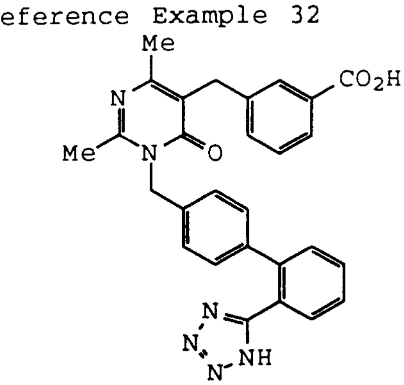

Reference Example 33

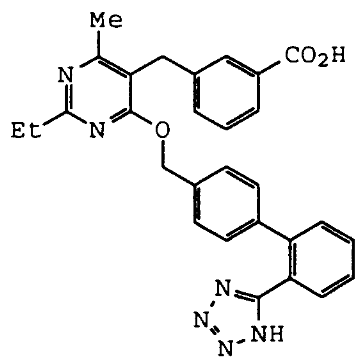

Reference Example 34

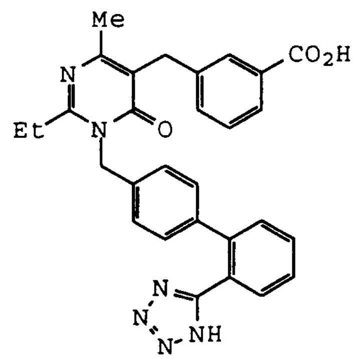

Reference Example 35

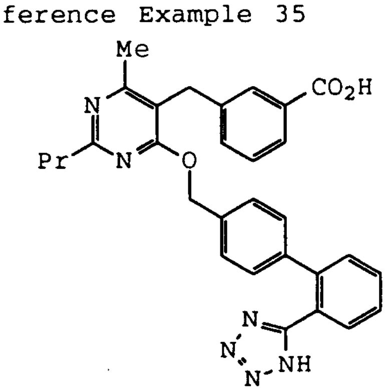

Reference Example 36

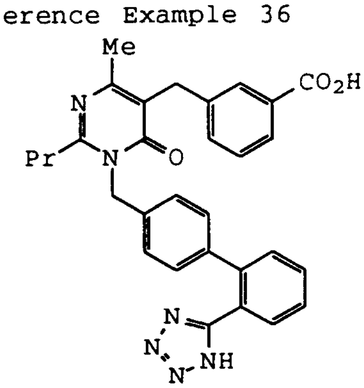

Reference Example 37

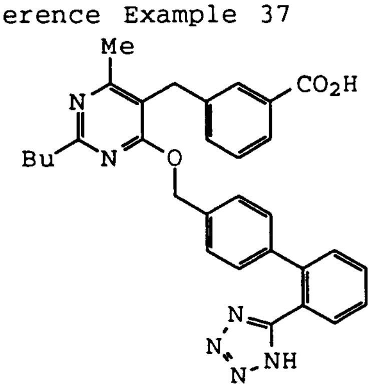

Reference Example 38

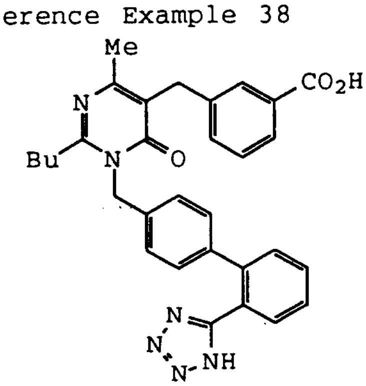

Reference Example 39

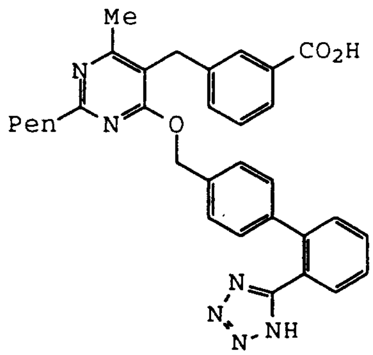

Reference Example 40

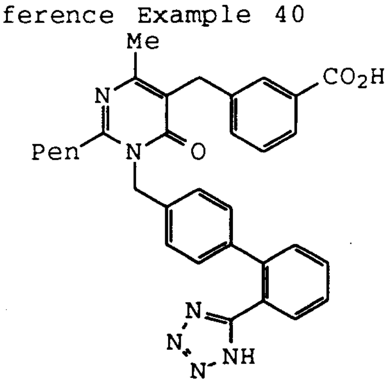

Reference Example 41

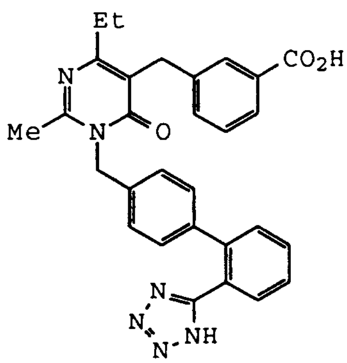

Reference Example 42

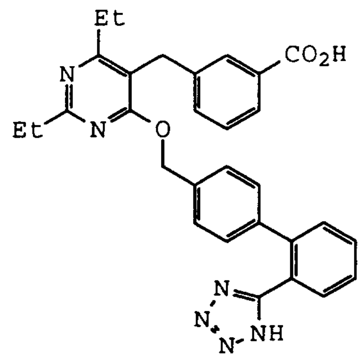

Reference Example 43

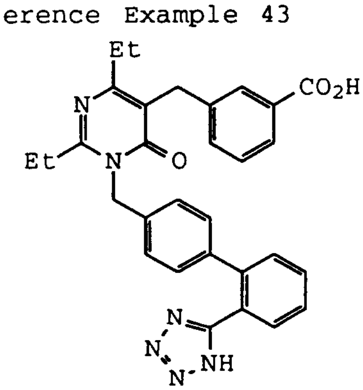

Reference Example 44

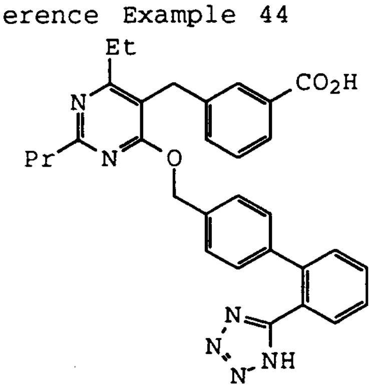

Reference Example 45

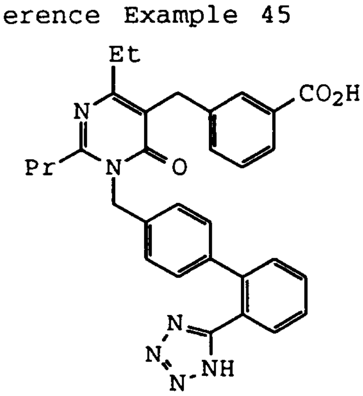

Reference Example 46

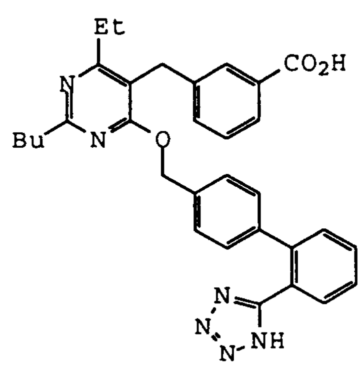

Reference Example 47

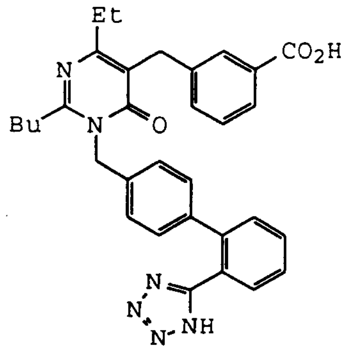

Reference Example 48

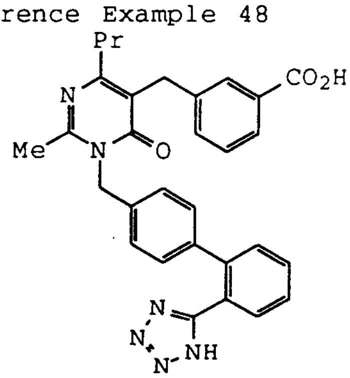

Reference Example 49

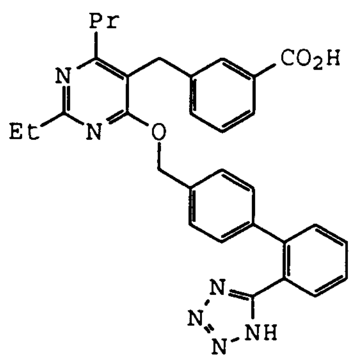

Reference Example 50

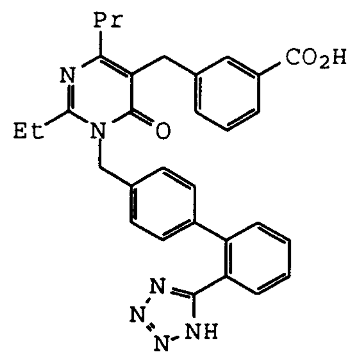

Reference Example 51

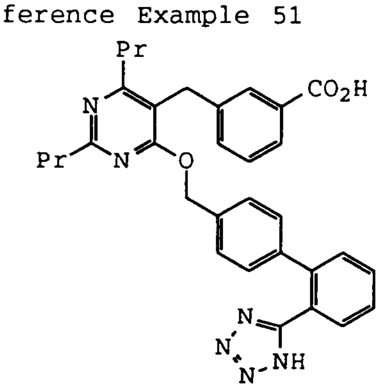

Reference Example 52

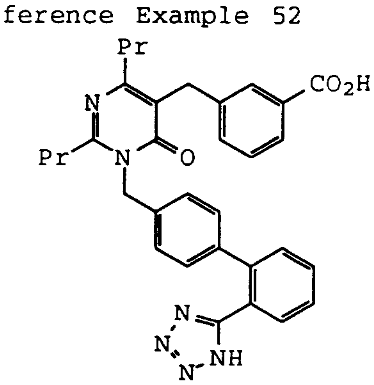

Reference Example 53

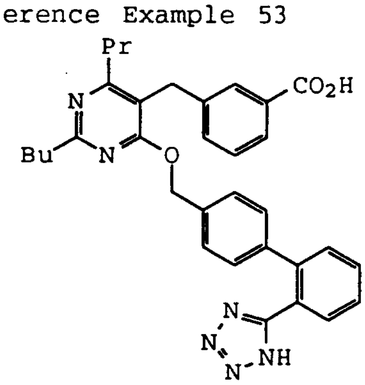

Reference Example 54

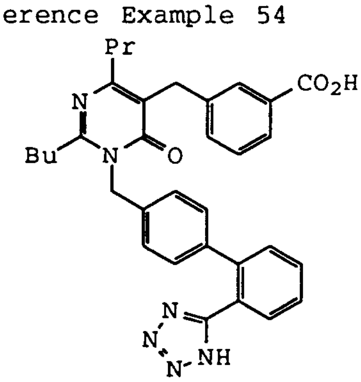

Reference Example 55

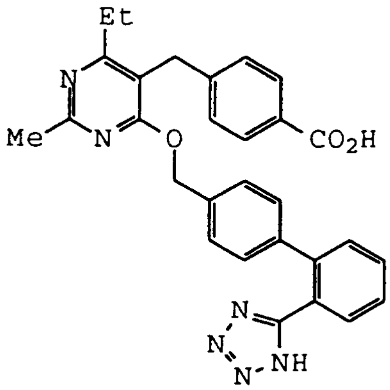

Reference Example 56

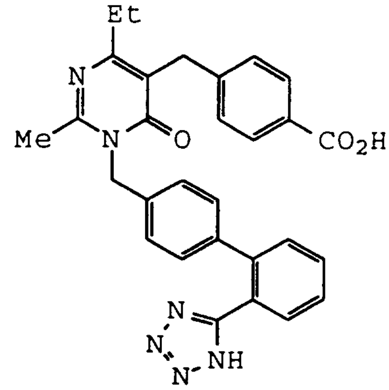

Reference Example 57

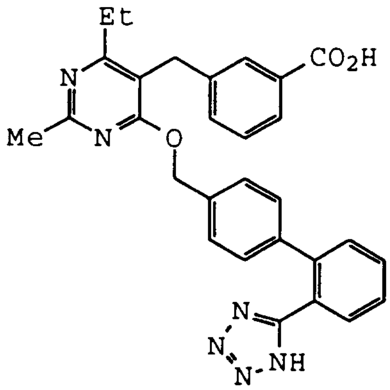

Reference Example 58

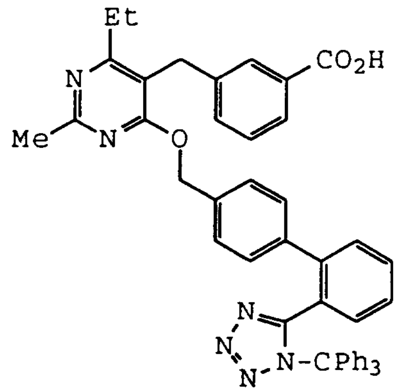

## Claims

1. A pyrimidine derivative of the general formula (I):

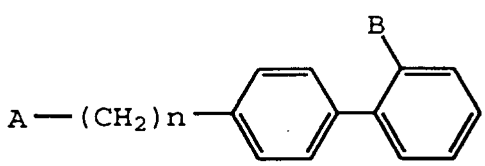

(I)

wherein A is a group of the general formula (I-1):

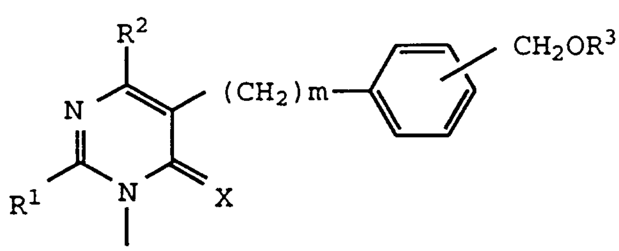

(I-1)

wherein $R^1$ and $R^2$, which may be same or different, are a hydrogen or halogen atom, lower alkyl, lower alkenyl, lower alkynyl, lower alkoxy, lower alkylthio, acetyl, phenyl, or substituted phenyl group, $R^3$ is a hydrogen atom, lower alkyl, lower alkenyl, lower alkynyl, or acetyl group, X is O, NH, or $S(O)_p$, p is an integer of 0 to 2, and m is an integer of 1 to 2, or a group of the general formula (I-2):

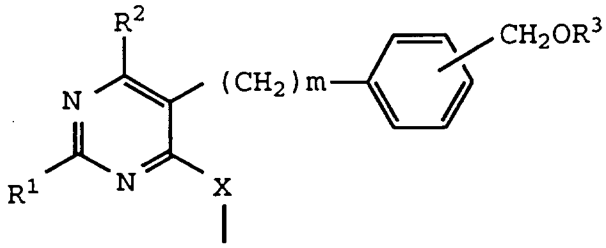

(I-2)

wherein $R^1$, $R^2$, $R^3$, X, p, and m have the same meanings as above, B is a carboxyl, lower alkoxycarbonyl, cyano, tetrazolyl, or protected tetrazolyl group, and n is an integer of 1 to 2, or a salt thereof.

**2.** A pyrimidine derivative according to claim 1 of the general formula (I) wherein $R^1$ is a lower alkyl group of 1 to 6 carbon atoms, $R^2$ is a lower alkyl group of 1 to 4 carbon atoms, $R^3$ is a hydrogen atom, a lower alkyl group of 1 to 2 carbon atoms, or a lower alkanoyl group of 2 to 4 carbon atoms, X is O, m is 1, B is a cyano, tetrazolyl, or a protected tetrazolyl group, and n is 1, or a salt thereof.

**3.** A pyrimidine derivative according to claim 1, wherein $R^1$ is a methyl, ethyl, propyl, butyl, or pentyl group, $R^2$ is a methyl, ethyl, or propyl group, $R^3$ is a hydrogen atom, methyl, or acetyl group, X is O, m is 1, B is a cyano, tetrazolyl, or trityl tetrazolyl group, and n is 1, or a salt thereof.

**4.** A process for manufacturing a pyrimidine derivative of the general formula (I):

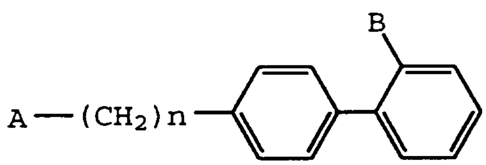

(I)

wherein A is a group of the general formula (I-1):

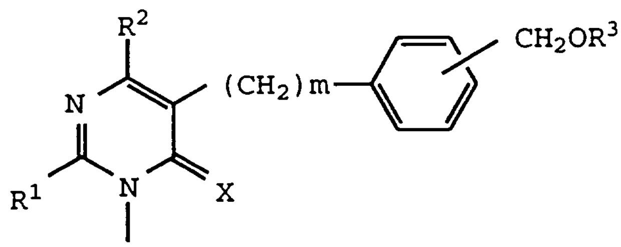

(I-1)

wherein $R^1$ and $R^2$, which may be same or different, are a hydrogen or halogen atom, lower alkyl, lower alkenyl, lower alkynyl, lower alkoxy, lower alkylthio, acetyl, phenyl, or substituted phenyl group, $R^3$ is a hydrogen atom, lower alkyl, lower alkenyl, lower alkynyl, or acetyl group, X is O, NH, or $S(O)_p$, p is an integer of 0 to 2, and m is an integer of 1 to 2, or a group of the general formula (I-2):

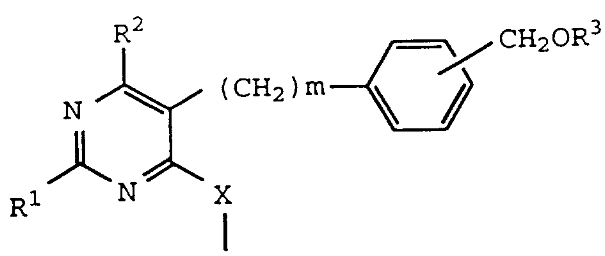

(I-2)

wherein $R^1$, $R^2$, $R^3$, X, and m have the same meanings as above, B is a carboxyl, lower alkoxycarbonyl, cyano, tetrazolyl, or protected tetrazolyl group, and n is an integer of 1 to 2, or a salt thereof, characterized by reacting, in the presence of a base, a compound of the general formula (II):

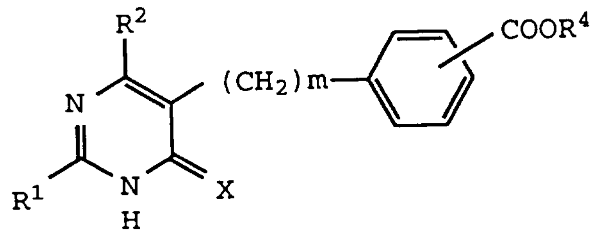

(II)

wherein $R^1$, $R^2$, X, and m have the same meanings as the above, and $R^4$ is a hydrogen atom, lower alkyl, or substituted lower alkyl group, and a compound of the general formula (III):

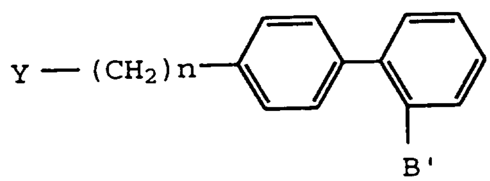

(III)

wherein B' is a lower alkoxycarbonyl, cyano, or protected tetrazolyl group, Y is a halogen atom, alkylsulfonyloxy, or arylsulfonyloxy group, and n has the same meaning as the above, to form a compound of the general formula (IV):

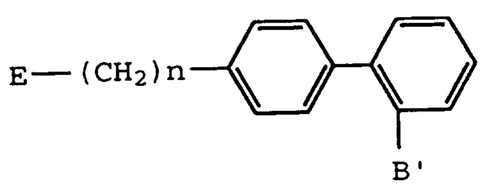

(IV)

wherein E is a group of the general formula (IV-1):

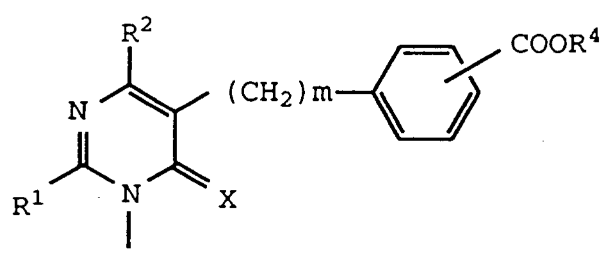

(IV-1)

wherein $R^1$, $R^2$, $R^4$, X, and m have the same meanings as the above, or a group of the general formula (IV-2):

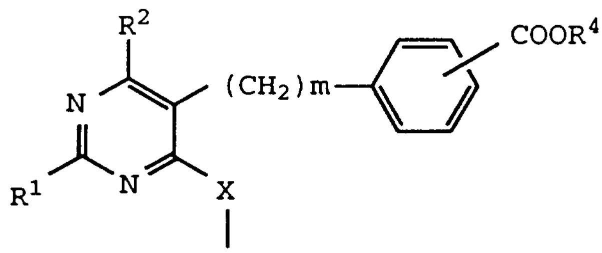

(IV-2)

wherein $R^1$, $R^2$, $R^4$, X, and m have the same meanings as the above, and B' and n have the same meanings as the above, reducing the resulting compound of the general formula (IV) to convert $-COOR^4$ in the above general formulae (IV-1) and (IV-2) into $-CH_2OH$, and optionally treating with an alkylating, alkenylating, alkynylating or acylating agent.

**5.** A process for manufacturing a pyrimidine derivative of the general formula (Id):

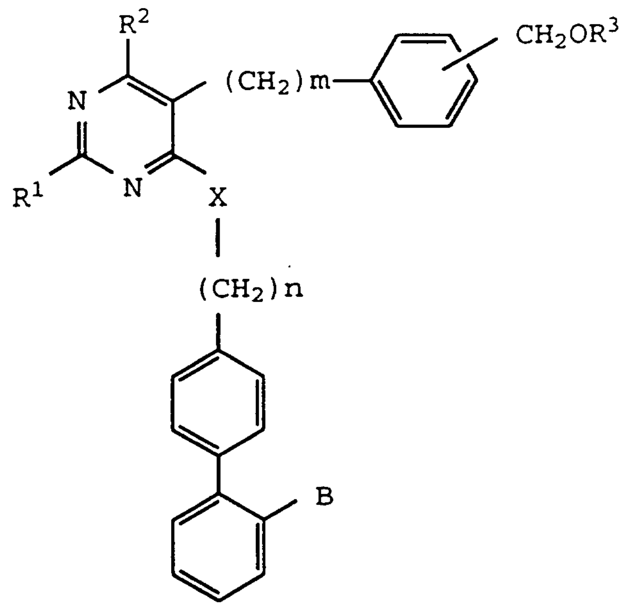

(Id)

wherein $R^1$ and $R^2$, which may be same or different, are a hydrogen or halogen atom, lower alkyl, lower alkenyl, lower alkynyl, lower alkoxy, lower alkylthio, acetyl, phenyl, or substituted phenyl group, $R^3$ is a hydrogen atom, lower alkyl, lower alkenyl, lower alkynyl, or acetyl group, B is a carboxyl, lower alkoxycarbonyl, cyano, tetrazolyl, or protected tetrazolyl group, m is an integer of 1 to 2, and n is an integer of 1 to 2, or a salt thereof, characterized by reacting a compound of the general formula (VII):

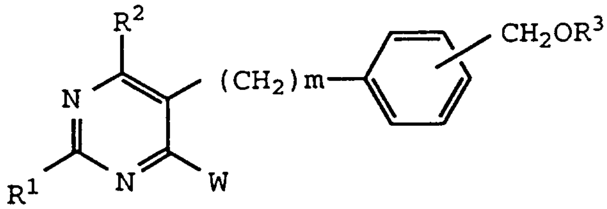

(VII)

wherein $R^1$, $R^2$, $R^3$, and m have the same meanings as above, and W is a halogen atom or nitro group, and a compound of the general formula (VIII):

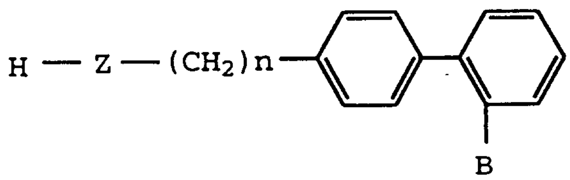

(VIII)

wherein B and n have the same meanings as above, and Z is O, NH or S, and optionally converting $R^3$ and/or B into other $R^3$ and/or B.

**6.** A pharmaceutical composition characterized by containing a pyrimidine derivative of the general formula (I):

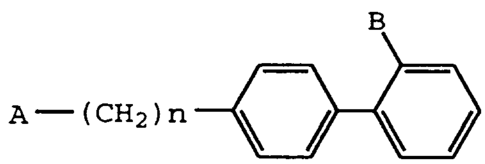

(I)

wherein A is a group of the general formula (I-1):

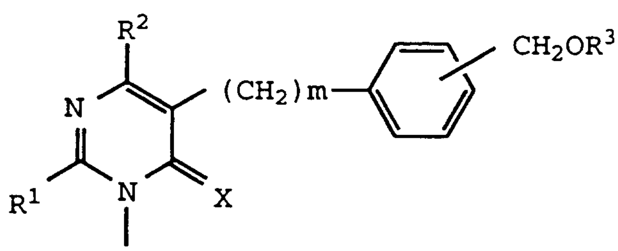

(I-1)

wherein $R^1$ and $R^2$, which may be same or different, are a hydrogen or halogen atom, lower alkyl, lower alkenyl, lower alkynyl, lower alkoxy, lower alkylthio, acetyl, phenyl, or substituted phenyl group, $R^3$ is a hydrogen atom, lower alkyl, lower alkenyl, lower alkynyl, or acetyl group, X is O, NH, or $S(O)_p$, p is an integer of 0 to 2, and m is an integer of 1 to 2, or a group of the general formula (I-2):

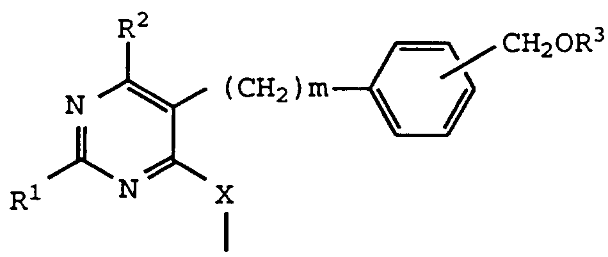

(I-2)

wherein $R^1$, $R^2$, $R^3$, X, p, and m have the same meanings as above, B is a carboxyl, lower alkoxycarbonyl, cyano, tetrazolyl, or protected tetrazolyl group, and n is an integer of 1 to 2, or a pharmaceutically acceptable salt thereof, and a pharmaceutically acceptable carrier.

**7.** A pharmaceutical composition according to claim 6, which is an angiotensin II antagonist.

**8.** A pharmaceutical composition according to claim 6, which is an agent for treating cardiovascular diseases.

**9.** Use of the compound according to claim 1 for preparing a pharmaceutical composition.

**10.** A method for treatment of angiotensin II inhibition comprising administering to a mammal in need thereof the compound according to claim 1 in an amount effective for angiotensin II inhibition.

## INTERNATIONAL SEARCH REPORT

International application No.
PCT/JP94/02114

**A. CLASSIFICATION OF SUBJECT MATTER**

Int. Cl[6] C07D239/34, C07D239/36, C07D239/38, C07D239/40, C07D239/42, C07D403/12, A61K31/505

According to International Patent Classification (IPC) or to both national classification and IPC

**B. FIELDS SEARCHED**

Minimum documentation searched (classification system followed by classification symbols)

Int. Cl[6] C07D239/34, C07D239/36, C07D239/38, C07D239/40, C07D239/42, C07D403/12, A61K31/505

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | US, A, 5087634 (G. D. Searle & Co.), February 11, 1992 (11. 02. 92), ABSTRACT & WO, A, 9207834 | 1-10 |
| A | EP, A, 445811 (TAKEDA CHEMICAL INDUSTRIES, LTD.), September 11, 1991 (11. 09. 91), whole document &JP, A, 5-155862 | 1-10 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| February 8, 1995 (08. 02. 95) | February 28, 1995 (28. 02. 95) |

| Name and mailing address of the ISA/ | Authorized officer |
|---|---|
| Japanese Patent Office | |
| Facsimile No. | Telephone No. |